# EUROPEAN PATENT APPLICATION

(11) **EP 2 330 190 A1**
(43) Date of publication of application: **08.06.2011**
(21) Application number: 09810088.6
(22) Date of filing: 01.09.2009
(51) Int. Cl.: C12N 9/06, C12P 13/00, C12P 17/10, C12N 15/09, C12R 1/465

(54) **PROCESS FOR PRODUCTION OF OPTICALLY ACTIVE AMINE DERIVATIVE**

(30) Priority: 01.09.2008 JP 2008223625
(71) Applicant: Daicel Chemical Industries, Ltd., Osaka-shi, Osaka 530-0001 (JP)
(72) Inventor: NAGASAWA, Toru, Gifu-shi Gifu 501-1174 (JP); YOSHIDA, Toyokazu, Gifu-shi Gifu 501-1121 (JP); ISHIDA, Koichi, Gifu-shi Gifu 501-1131 (JP); YAMAMOTO, Hiroaki, Himeji-shi Hyogo 671-1281 (JP); KIMOTO, Norihiro, Himeji-shi Hyogo 671-1281 (JP)
(74) Representative: Grünecker, Kinkeldey, Stockmair & Schwanhäusser Anwaltssozietät
(86) International application number: PCT/JP2009/065240
(87) International publication number: WO 2010/024444

(57) **Abstract**

Novel enzymes that stereoselectively reduce imine derivatives were isolated and purified, and polynucleotides encoding the enzymes were cloned. Optically active amine derivatives were produced by acting on imine derivatives with a culture of microorganisms having the ability to stereoselectively reduce the compounds, microbial cells or processed products thereof, and/or imine reductases thereof, followed by collecting the generated optically active amine derivatives. The present invention enables, for example, production of an optically active compound represented by formula (IV): 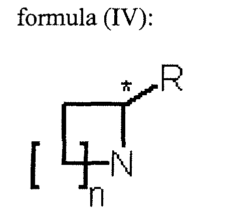 (wherein R group represents an alkyl group having one to three carbon atoms; and n represents an integer of 1 to 4).

SEQUENCE LISTING

<110> DAICEL CHEMICAL INDUSTRIES, LTD.

<120> Process for Production of Optically Active Amine Derivative

<130> EP75136FZapu

<140> 09 810 088.6
<141> 2009-09-01

<150> PCT/JP2009/065240
<151> 2009-09-01

<150> JP 2008-223625
<151> 2008-09-01

<160> 19

<170> PatentIn version 3.4

<210> 1
<211> 1486
<212> DNA
<213> Streptomyces sp. GF3587

<400> 1
gagtttgatc ctggctcagg acgaacgctg gcggcgtgct taacacatgc aagtcgaacg 60

atgaagcctt tcggggtgga ttagtggcga acgggtgagt aacacgtggg caatctgccc 120

ttcactctgg gacaagccct ggaaacgggg tctaataccg gataacactc tgtcctgcat 180

gggacggggt taaaagctcc ggcggtgaag gatgagcccg cggcctatca gcttgttggt 240

ggggtaatgg cctaccaagg cgacgacggg tagccggcct gagagggcga ccggccacac 300

tgggactgag acacggccca gactcctacg ggaggcagca gtggggaata ttgcacaatg 360

ggcgaaagcc tgatgcagcg acgccgcgtg agggatgacg gccttcgggt tgtaaacctc 420

tttcagcagg gaagaagcga aagtgacggt acctgcagaa gaagcgccgg ctaactacgt 480

gccagcagcc gcggtaatac gtagggcgca agcgttgtcc ggaattattg ggcgtaaaga 540

gctcgtaggc ggcttgtcac gtcggatgtg aaagcccggg gcttaacccc gggtctgcat 600

tcgatacggg ctagctagag tgtggtaggg gagatcggaa ttcctggtgt agcggtgaaa 660

tgcgcagata tcaggaggaa caccggtggc gaaggcggat ctctgggcca ttactgacgc 720

tgaggagcga aagcgtgggg agcgaacagg attagatacc ctggtagtcc acgccgtaaa 780

cgttgggaac taggtgttgg cgacattcca cgtcgtcggt gccgcagcta acgcattaag 840

ttccccgcct ggggagtacg gccgcaaggc taaaactcaa aggaattgac gggggcccgc 900

acaagcagcg gagcatgtgg cttaattcga cgcaacgcga agaaccttac caaggcttga 960

catataccgg aaagcatcag agatggtgcc ccccttgtgg tcggtataca ggtggtgcat 1020

ggctgtcgtc agctcgtgtc gtgagatgtt gggttaagtc ccgcaacgag cgcaaccctt 1080

gttctgtgtt gccagcatgc ccttcggggt gatggggact cacaggagac tgccggggtc 1140

aactcggagg aaggtgggga cgacgtcaag tcatcatgcc ccttatgtct tgggctgcac 1200

acgtgctaca atggccggta caatgagctg cgatgccgcg aggckgagcg aatctcaaaa 1260

agccggtctc agttcggatt ggggtctgca actcgacccc atgaagtcgg agttgctagt 1320

aatcgcagat cagcattgct gcggtgaata cgttcccggg ccttgtacac accgcccgtc 1380

acgtcacgaa agtcggtaac acccgaagcc ggtggcccaa ccccttgtgg gagggagctg 1440

tcgaaggtgg gactggcgat tgggacgaag tcgtaacaag gtagcc 1486

<210> 2
<211> 502
<212> DNA
<213> Streptomyces sp. GF3501

<400> 2
tggagagttt gatcctggct caggacgaac gctggcggcg tgcttaacac atgcaagtcg 60

aacgatgaac cacttcggtg gggattagtg gcgaacgggt gagtaacacg tgggcaatct 120

gcccttcact ctgggacaag ccctggaaac ggggtctaat accggataac acttccactc 180

tcctgggtgg aggttaaaag ctccggcggt gaaggatgag cccgcggcct atcagcttgt 240

tggtgaggta acggctcacc aaggcgacga cgggtagccg gcctgagagg gcgaccggcc 300

acactgggac tgagacacgg cccagactcc tacgggaggc agcagtgggg aatattgcac 360

aatgggcgaa agcctgatgc agcgacgccg cgtgagggat gacggccttc gggttgtaaa 420

cctctttcag cagggaagaa gcgaaagtga cggtacctgc agaagaagcg ccggctaact 480

acgtgccagc agccgcggta at 502

<210> 3
<211> 502
<212> DNA
<213> Streptomyces sp. GF3585

<400> 3
tggagagttt gatcctggct caggacgaac gctggcggcg tgcttaacac atgcaagtcg 60

aacgatgaag cctttcgggg tggattagtg gcgaacgggt gagtaacacg tgggcaatct 120

gcccttcact ctgggacaag ccctggaaac ggggtctaat accggataac actctgtccc 180

gcatgggacg gggttaaaag ctccggcggt gaaggatgag cccgcggcct atcagcttgt 240

tggtggggta atggcctacc aaggcgacga cgggtagccg gcctgagagg gcgaccggcc 300

acactgggac tgagacacgg cccagactcc tacgggaggc agcagtgggg aatattgcac 360

aatgggcgaa agcctgatgc agcgacgccg cgtgagggat gacggccttc gggttgtaaa 420

cctctttcag cagggaagaa gcgaaagtga cggtacctgc agaagaagcg ccggctaact 480

acgtgccagc agccgcggta at 502

<210> 4
<211> 502
<212> DNA
<213> Streptomyces sp. GF4415

<400> 4
tggagagttt gatcctggct caggacgaac gctggcggcg tgcttaacac atgcaagtcg 60

aacgatgaag cccttcgggg tggattagtg gcgaacgggt gagtaacacg tgggcaatct 120

gcccttcact ctgggacaag ccctggaaac ggggtctaat accggatams asyksskkms 180

gcatskssss krstkgaaag ctccggcggt gaaggatgag cccgcggcct atcagcttgt 240

tggtggggta atggcctacc aaggcgacga cgggtagccg gcctgagagg gcgaccggcc 300

acactgggac tgagacacgg cccagactcc tacgggaggc agcagtgggg aatattgcac 360

aatgggcgaa agcctgatgc agcgacgccg cgtgagggat gacggccttc gggttgtaaa 420

cctctttcag cagggaagaa gcgagagtga cggtacctgc agaagaagcg ccggctaact 480

acgtgccagc agccgcggta at 502

<210> 5
<211> 888
<212> DNA
<213> Streptomyces sp. GF3587

<400> 5
atgggcgaca accgcacccc ggtgacggtc atcggcctcg gcctcatggg acaggcgctc 60

gccgccgcgt tcctcgaggc cggccacacc acgaccgtgt ggaaccgttc ggcgggcaag 120

gccgagcagc tcgtctcgca gggcgcggtg caggccgcca cgcccgccga cgcggtggcc 180

gccagtgagc tggtcgtggt ctgcctgtcg acgtacgaca acatgcacga cgtgatcggt 240

tcgctcggtg agtccctccg gggcaaggtc atcgtgaacc tgacgtccgg ctcgtcggac 300

cagggccgcg agacggcggc gtgggccgag aagcagggcg tggagtacct cgacggcgcg 360

atcatgatca cgccgcccgg catcggcacc gagaccgccg tcctcttcta cgccggtacg 420

cagagcgtgt tcgagaagta cgagcccgcg ctcaagctgc tcggcggcgg caccacgtac 480

ctcggcaccg accacggtat gcccgcgctg tacgacgtgt cgctgctcgg cctcatgtgg 540

ggcacgctca acagcttcct gcacggtgtc gccgtggtgg agaccgcggg cgtcggcgcc 600

cagcagttcc tgccgtgggc gcacatgtgg ctggaggcga tcaagatgtt caccgccgac 660

tacgcggccc agatcgacgc cggtgacggc aagttccccg ccaacgacgc cacgctggag 720

acgcacctgg cggcgctcaa gcacctggtg cacgagagcg aggcgctggg tatcgacgcc 780

gaactgccga agtactccga ggcgttgatg gagcgcgtca tcagccaggg tcacgccaag 840

aacagctacg cggccgtgct caaggcgttc cgcaagccgt cggagtga 888

<210> 6
<211> 295
<212> PRT
<213> Streptomyces sp. GF3587

<400> 6

Met Gly Asp Asn Arg Thr Pro Val Thr Val Ile Gly Leu Gly Leu Met
1               5                   10                  15

Gly Gln Ala Leu Ala Ala Ala Phe Leu Glu Ala Gly His Thr Thr Thr
            20                  25                  30

Val Trp Asn Arg Ser Ala Gly Lys Ala Glu Gln Leu Val Ser Gln Gly
        35                  40                  45

Ala Val Gln Ala Ala Thr Pro Ala Asp Ala Val Ala Ala Ser Glu Leu
    50                  55                  60

Val Val Val Cys Leu Ser Thr Tyr Asp Asn Met His Asp Val Ile Gly
65                  70                  75                  80

Ser Leu Gly Glu Ser Leu Arg Gly Lys Val Ile Val Asn Leu Thr Ser
                85                  90                  95

Gly Ser Ser Asp Gln Gly Arg Glu Thr Ala Ala Trp Ala Glu Lys Gln
            100                 105                 110

Gly Val Glu Tyr Leu Asp Gly Ala Ile Met Ile Thr Pro Pro Gly Ile
        115                 120                 125

Gly Thr Glu Thr Ala Val Leu Phe Tyr Ala Gly Thr Gln Ser Val Phe
    130                 135                 140

Glu Lys Tyr Glu Pro Ala Leu Lys Leu Leu Gly Gly Gly Thr Thr Tyr
145                 150                 155                 160

Leu Gly Thr Asp His Gly Met Pro Ala Leu Tyr Asp Val Ser Leu Leu
                165                 170                 175

Gly Leu Met Trp Gly Thr Leu Asn Ser Phe Leu His Gly Val Ala Val
            180                 185                 190

Val Glu Thr Ala Gly Val Gly Ala Gln Gln Phe Leu Pro Trp Ala His
        195                 200                 205

Met Trp Leu Glu Ala Ile Lys Met Phe Thr Ala Asp Tyr Ala Ala Gln
    210                 215                 220

Ile Asp Ala Gly Asp Gly Lys Phe Pro Ala Asn Asp Ala Thr Leu Glu
225                 230                 235                 240

Thr His Leu Ala Ala Leu Lys His Leu Val His Glu Ser Glu Ala Leu
                245                 250                 255

Gly Ile Asp Ala Glu Leu Pro Lys Tyr Ser Glu Ala Leu Met Glu Arg
            260                 265                 270

Val Ile Ser Gln Gly His Ala Lys Asn Ser Tyr Ala Ala Val Leu Lys
        275                 280                 285

Ala Phe Arg Lys Pro Ser Glu
    290                 295

<210>  7
<211>  30
<212>  PRT
<213>  Streptomyces sp. GF3587

<400>  7

Gly Asp Asn Arg Thr Pro Val Thr Val Ile Gly Leu Gly Leu Met Gly
1               5                   10                  15

Gln Ala Leu Ala Ala Ala Phe Leu Glu Ala Gly Phe Thr Thr
            20                  25                  30

<210>  8
<211>  16
<212>  PRT
<213>  Streptomyces sp. GF3587

<400>  8

His Leu Val His Glu Ser Glu Ala Leu Gly Ile Asp Ala Glu Leu Pro
1               5                   10                  15

<210>  9
<211>  34
<212>  DNA
<213>  Artificial

<220>
<223>  An artificially synthesized primer sequence

<220>
<221>  misc_feature
<222>  (32)..(32)
<223>  n is a, c, g, or t

<400>  9
gacgaattct aggsgacaac mgsacsccsg tnac                                34

<210>  10
<211>  35
<212>  DNA
<213>  Artificial

<220>
<223>  An artificially synthesized primer sequence

<400>  10
gacgaattct actcsgcgtc gatsccsars gcytc                               35

<210>  11
<211>  694
<212>  DNA
<213>  Streptomyces sp. GF3587

<400>  11
tgggacaggc gctcgccgcc gcgttcctcg aggccggcca caccacgacc gtgtggaacc      60

gttcggcggg caaggccgag cagctcgtct cgcagggcgc ggtgcaggcc gccacgcccg     120

ccgacgcggt ggccgccagt gagctggtcg tggtctgcct gtcgacgtac gacaacatgc     180

acgacgtgat cggttcgctc ggtgagtccc tccggggcaa ggtcatcgtg aacctgacgt 240

ccggctcgtc ggaccagggc cgcgagacgg cggcgtgggc cgagaagcag ggcgtggagt 300

acctcgacgg cgcgatcatg atcacgccgc ccggcatcgg caccgagacc gccgtcctct 360

tctacgccgg tacgcagagc gtgttcgaga agtacgagcc cgcgctcaag ctgctcggcg 420

gcggcaccac gtacctcggc accgaccacg gtatgcccgc gctgtacgac gtgtcgctgc 480

tcggcctcat gtggggcacg ctcaacagct tcctgcacgg tgtcgccgtg gtggagaccg 540

cgggcgtcgg cgcccagcag ttcctgccgt gggcgcacat gtggctggag gcgatcaaga 600

tgttcaccgc cgactacgcg gcccagatcg acgccggtga cggcaagttc cccgccaacg 660

acgccacgct ggagacgcac ctggcggcgc tcaa 694

<210> 12
<211> 23
<212> DNA
<213> Artificial

<220>
<223> An artificially synthesized primer sequence

<400> 12
tgcatgttgt cgtacgtcga cag 23

<210> 13
<211> 23
<212> DNA
<213> Artificial

<220>
<223> An artificially synthesized primer sequence

<400> 13
ctggaggcga tcaagatgtt cac 23

<210> 14
<211> 29
<212> DNA
<213> Artificial

<220>
<223> An artificially synthesized primer sequence

<400> 14
catccatggg tgataatcgc accccggtg 29

<210> 15
<211> 37
<212> DNA
<213> Artificial

<220>
<223> An artificially synthesized primer sequence

<400> 15
ctgtctagat tattcagacg gcttgcggaa cgccttg 37

<210>  16
<211>  930
<212>  DNA
<213>  Streptomyces kanamyceticus

<400>  16
atgcccgaca acccctctac gaaagggcgc atgatgcgga accagcaggc agagcacacc      60

cctgtgacgg tcatcggcct cggcctcatg ggccaggccc tcgccggggc gttcctgggc     120

gcgggacacc ccacgaccgt gtggaaccgg acggcggcga aggccgaacc gctcgtcgcc     180

cggggcgcga agagcgcggg atcggtcgcc gaagcggtcg ccgcgagccc gctggtggtc     240

gtctgcgtct cggactacga cgccgtgcac gcgctcctcg acccgctcga cggcacggcc     300

ctccaggggc gcaccctggt gaacctgacc tccggcacct cggcccaggc ccgcgagcgc     360

gccgcgtggg cggacggccg cggcgccgac tacctcgacg gcgcgatcct ggcgggaccc     420

gccgcgatcg gcacggcgga cgccgtcgtc ctcctcagcg ggccccggtc ggccttcgac     480

ccgcacgcgt ctgccctcgg cggcctcggc gcgggaacga cgtacctcgg cgccgaccac     540

ggtctcgcgt cgctgtacga cgcggcgggc ctcgtcatga tgtggagcat cctgaacggc     600

ttcctgcaag gcgccgcgct gctcggcacg gccggtgtgg acgccacgac gttcgccccg     660

ttcatcaccc agggaatagg aaccgtcgcc gactggttgc ccggctacgc gcgacagatc     720

gacgacggcg cgtaccccgc cgacgacgcc gccatcgaca cccatctggc caccatggaa     780

cacctcatcc acgagagcga gttcctcgga gtgaacgccg aactccccag gttcatcaag     840

gccctcgccg accgggccgt ggccgacggg cacggcggca gcggctaccc ggcgctgatc     900

gaacagttcc gcacgcactc agggaagtga                                      930

<210>  17
<211>  309
<212>  PRT
<213>  Streptomyces kanamyceticus

<400>  17

Met Pro Asp Asn Pro Ser Thr Lys Gly Arg Met Met Arg Asn Gln Gln
1               5                   10                  15

Ala Glu His Thr Pro Val Thr Val Ile Gly Leu Gly Leu Met Gly Gln
            20                  25                  30

Ala Leu Ala Gly Ala Phe Leu Gly Ala Gly His Pro Thr Thr Val Trp
        35                  40                  45

Asn Arg Thr Ala Ala Lys Ala Glu Pro Leu Val Ala Arg Gly Ala Lys
    50                  55                  60

Ser Ala Gly Ser Val Ala Glu Ala Val Ala Ala Ser Pro Leu Val Val
65                  70                  75                  80

Val Cys Val Ser Asp Tyr Asp Ala Val His Ala Leu Leu Asp Pro Leu
                85                  90                  95

Asp Gly Thr Ala Leu Gln Gly Arg Thr Leu Val Asn Leu Thr Ser Gly
            100                 105                 110

Thr Ser Ala Gln Ala Arg Glu Arg Ala Ala Trp Ala Asp Gly Arg Gly
        115                 120                 125

Ala Asp Tyr Leu Asp Gly Ala Ile Leu Ala Gly Pro Ala Ala Ile Gly
    130                 135                 140

Thr Ala Asp Ala Val Val Leu Leu Ser Gly Pro Arg Ser Ala Phe Asp
145                 150                 155                 160

Pro His Ala Ser Ala Leu Gly Gly Leu Gly Ala Gly Thr Thr Tyr Leu
                165                 170                 175

Gly Ala Asp His Gly Leu Ala Ser Leu Tyr Asp Ala Ala Gly Leu Val
            180                 185                 190

Met Met Trp Ser Ile Leu Asn Gly Phe Leu Gln Gly Ala Ala Leu Leu
        195                 200                 205

Gly Thr Ala Gly Val Asp Ala Thr Thr Phe Ala Pro Phe Ile Thr Gln
    210                 215                 220

Gly Ile Gly Thr Val Ala Asp Trp Leu Pro Gly Tyr Ala Arg Gln Ile
225                 230                 235                 240

Asp Asp Gly Ala Tyr Pro Ala Asp Asp Ala Ala Ile Asp Thr His Leu
                245                 250                 255

Ala Thr Met Glu His Leu Ile His Glu Ser Glu Phe Leu Gly Val Asn
            260                 265                 270

Ala Glu Leu Pro Arg Phe Ile Lys Ala Leu Ala Asp Arg Ala Val Ala
        275                 280                 285

Asp Gly His Gly Gly Ser Gly Tyr Pro Ala Leu Ile Glu Gln Phe Arg
    290                 295                 300

Thr His Ser Gly Lys
305

<210>  18
<211>  35
<212>  DNA
<213>  Artificial

<220>
<223>  An artificially synthesized primer sequence

<400>  18
gaggaataat ccatgcccga caacccctct acgaa                                35

<210>  19
<211>  36
<212>  DNA
<213>  Artificial

<220>
<223>  An artificially synthesized primer sequence

<400>  19
tatgaattcc tctagattac ttccctgagt gcgtgc                                36

## Description

### Technical Field

The present invention relates to methods for producing optically active amine derivatives to be used as raw materials or intermediates for synthesizing pharmaceuticals or the like by stereoselectively reducing imine derivatives.

### Background Art

Enzymes involved in amino acid metabolism, such as 1-pyrroline-2-carboxylate or 1-pyrroline-5-carboxylate, and enzymes that reduce imine derivatives having a special structure and function, such as dihydrofolate reductase, are known to those skilled in the art. However, other than the reports listed below, little is known about enzymes or microorganisms that produce optically active amine derivatives by stereoselectively reducing simple imine compounds such as 2-methyl-1-pyrroline.
Non-patent Document 1: Tetrahedron, 60, 753-758, 2004
Reduction of N-benzylidenebenzylamine by *Acetobacterium woodi*
Non-patent Document 2: Tetrahedron Asymmetry, 19, 93-96 (2008)
Reduction of N-((E)-N-(1-phenylethylidene) aniline derivatives by *Candida parapsilosis*

However, since the reduced product of N-benzylidenebenzylamine has no chirality, the stereoselectivity of the reaction is unclear. Furthermore, the reduction of N-((E)-N-(1-phenylethylidene) aniline derivatives by *Candida parapsilosis* is very inefficient and thus is not suitable for industrial application. In addition, the enzymes responsible for the imine reduction remain unidentified.

On the other hand, the following methods are known for producing 2-methylpyrrolidine:
Non-patent Document 3: Acta. Pharm. Suec., 15, 255-263, 1978.

Method for producing chiral 2-methylpyrrolidine by optically resolving racemic 2-methylpyrrolidine using tartaric acid, in which racemic 2-methylpyrrolidine is produced by reducing 2-methyl-1-pyrroline with sodium borohydride.
Non-patent Document 4: J. Org. Chem., 54, 209-216, 1989.

Method for producing 2-methylpyrrolidine, in which the hydroxyl group of L-prolinol derived from L-proline is converted into a chloro group using thionyl chloride; the nitrogen atom is protected with a benzyloxycarbonyl group; and then
(R)-1-benzyloxycarbonyl-2-methylpyrrolidine is produced via radical-based reduction using tributyltin hydride, followed by deprotection.
Patent Document 1: WO2005/073388

Method for producing (R)-2-methylpyrrolidine, in which optically active (S)-1,4-pentanediol is converted into disulfonate and cyclized by reaction with benzylamine to synthesize (R)-1-benzyl-2-methyl-1-pyrrolidine, followed by debenzylation.

However, all of these methods have the following problems:
- the process is long;
- dangerous hydride reagents are used;
- expensive optically active diols are used as raw materials; and
- the yield does not exceed 50% since the product is produced by an optical resolution method

### [Prior Art Documents]

### [Non-patent Documents]

[Non-patent Document 1] Tetrahedron, 60, 753-758 (2004)
[Non-patent Document 2] Tetrahedron Asymmetry, 19, 93-96 (2008)
[Non-patent Document 3] Acta. Pharm. Suec., 15, 255-263 (1978)
[Non-patent Document 4] J. Org. Chem., 54, 209-216 (1989)
[Patent Document 1] WO2005/073388

### Disclosure of the Invention

### [Problems to be Solved by the Invention]

An objective of the present invention is to provide methods for producing optically active amine derivatives from imine derivatives. Another objective of the present invention is to provide microorganisms and enzymes that are able to produce optically active amine derivatives using imine derivatives as substrates. Still another objective of the present invention is to obtain recombinants by isolating DNAs encoding enzymes having the properties of interest. Yet another objective of the present invention is to provide methods for producing optically active amine derivatives using the recombinants.

### [Means for Solving the Problems]

The present inventors searched for microorganisms that use imine derivatives as substrates to produce optically active amine derivatives, and successfully discovered microorganisms having the desired activity. The present inventors identified the microorganisms found and confirmed that as listed below the microorganisms all belong to the genus *Streptomyces.* The microorganisms identified by the present inventors produce, for example, (R)-2-methylpyrrolidine via asymmetric reduction of 2-methyl-1-pyrroline.
*Streptomyces* sp. GF3587;
*Streptomyces* sp. GF3501;
*Streptomyces* sp. GF3585; and
*Streptomyces* sp. GF4415.

Of these microorganisms, *Streptomyces* sp. GF3587 which exhibits the strongest activity was used to analyze the properties of an enzyme that produces (R)-2-methylpyrrolidine via asymmetric reduction of 2-methyl-1-pyrroline. The enzyme was revealed to be an NADPH-dependent imine reductase. The present inventors also successfully purified the imine reductase by preparing cell-free extracts from a large-scale culture of GF3587, followed by the steps described below:
- ammonium sulfate fractionation;
- ion exchange chromatography using DEAE-Sephacel;
- hydrophobic chromatography using Phenylsepharose; and
- affinity chromatography using 2',5'-ADP Sepharose 4B.

Furthermore, the present inventors isolated the enzyme-encoding DNA, and created recombinant bacteria expressing the enzyme at high levels.

Meanwhile, identity search of the amino acid sequence of SEQ ID NO: 6 against SWISS-PROT was performed using the BLAST program, and a protein identical to the imine reductase of the present invention was found. Specifically, it is a putative protein named Q1EQE0 and identified by genomic analysis of *Streptomyces kanamyceticus.* The function of the protein is unknown. Recombinant bacteria expressing Q1EQE0 at high levels were prepared and assessed for the properties of Q1EQE0. The result showed that Q1EQE0 had imine-reducing activity.

As described above, there are known enzymes involved in biosynthesis that stereoselectively reduce imine derivatives. However, all of the known enzymes are involved in amino acid or folic acid metabolism.

Therefore, substrate specificity and activity required for industrial application cannot be expected when the enzymes are used to produce intermediates for pharmaceuticals or such. In particular, there is no available method for producing optically active cyclic alkyl amine derivatives by reducing cyclic alkyl imine derivatives such as 2-methyl-1-pyrroline.

Thus, it was a very surprising result to discover microorganisms that have substrate specificity and catalytic activity suitable for production of intermediates for pharmaceuticals or the like. More surprisingly, the ability to stereoselectively reduce imines of the microorganisms was sufficiently high and meets the requirements of industrial application. Furthermore, the present inventors isolated a DNA encoding the imine reductase and then produced recombinant bacteria expressing the enzyme at high levels, thereby completing the present invention.

Specifically, the present invention provides methods for producing optically active amines described below. The present invention also relates to microorganisms and imine reductases that are useful for the methods, polynucleotides comprising DNAs encoding such enzymes, methods for producing the enzymes, and uses thereof.
[1]
   An imine reductase having the physicochemical properties of (1) to (5) below:
   (1) activity: to generate (R)-2-methylpyrrolidine by reducing 2-methyl-1-pyrroline in a reduced nicotinamide adenine dinucleotide phosphate (hereinafter abbreviated as NADPH)-dependent manner;
   (2) coenzyme dependency: use NADPH as a coenzyme;
   (3) optimal pH: 6.0 to 7.0;
   (4) optimal temperature: 40 to 55°C; and
   (5) molecular weight: approximately 32,000 by sodium dodecyl sulfate polyacrylamide gel electrophoresis (hereinafter abbreviated as SDS-PAGE), and 65,000 to 70,000 by gel filtration.
[2]
   The imine reductase of [1], which is derived from a microorganism belonging to the genus *Streptomyces.*
[3]
   The imine reductase of [2], wherein the microorganism belonging to the genus *Streptomyces* is any one selected from the group consisting of:
   *Streptomyces* sp. NITE BP-594;
   *Streptomyces* sp. NITE BP-595:
   *Streptomyces* sp. NITE BP-596; and
   *Streptomyces* sp. NITE BP-597.
[4]
   The imine reductase of [2], wherein the microorganism belonging to the genus *Streptomyces* is any one selected from the group consisting of:
   *Streptomyces griseorubiginosus;*
   *Streptomyces microflavus;* and
   *Streptomyces alboviridis.*
[5]
   The imine reductase of [4], wherein *Streptomyces griseorubiginosus, Streptomyces microflavus,* and *Streptomyces alboviridis* are the following microbial strains:
   *Streptomyces griseorubiginosus* NBRC 13047;
   *Streptomyces microflavus* NBRC 13062; and
   *Streptomyces alboviridis* NBRC 13013, respectively.
[6]
   An imine reductase that has the physicochemical properties of (1) and (2) of [1], and which is encoded by the polynucleotide of any one of (a) to (e) below:
   (a) a polynucleotide comprising the nucleotide sequence of SEQ ID NO: 5;
   (b) a polynucleotide encoding a protein comprising the amino acid sequence of SEQ ID NO: 6;
   (c) a polynucleotide encoding a protein comprising an amino acid sequence with a substitution, deletion, insertion, and/or addition of one or more amino acids in the amino acid sequence of SEQ ID NO: 6;
   (d) a polynucleotide that hybridizes under a stringent condition to a DNA comprising the nucleotide sequence of SEQ ID NO: 5; and
   (e) a polynucleotide encoding an amino acid sequence with 80% or higher sequence identity to the amino acid sequence of SEQ ID NO: 6.
[7]
   An imine reductase that has the physicochemical properties of (1) and (2) of [1], which comprises a protein having the function of generating (R)-2-methylpyrrolidine with at least 80%ee or higher optical purity and is encoded by the polynucleotide of any one of (a) to (e) below:
   (a) a polynucleotide comprising the nucleotide sequence of SEQ ID NO: 16;
   (b) a polynucleotide encoding a protein comprising the amino acid sequence of SEQ ID NO: 17;
   (c) a polynucleotide encoding a protein comprising an amino acid sequence with a substitution, deletion, insertion, and/or addition of one or more amino acids in the amino acid sequence of SEQ ID NO: 17;
   (d) a polynucleotide that hybridizes under a stringent condition to a DNA comprising the nucleotide sequence of SEQ ID NO: 16; and
   (e) a polynucleotide encoding an amino acid sequence that has 80% or higher sequence identity to the amino acid sequence of SEQ ID NO: 17.
[8]
   A method for producing an optically active R-isomer amine derivative, which comprises the steps of:
   contacting an imine derivative represented by formula (I) with at least one enzymatically active material selected from the group consisting of:
   a culture of microorganism having the ability to stereoselectively reduce the compound; a microbial cell; and a processed product thereof; and
   collecting the optically active R-isomer amine derivative represented by formula (II): 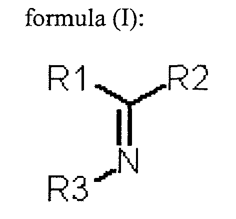 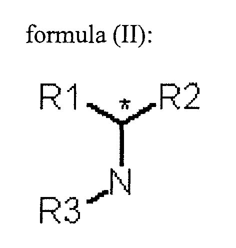 (wherein R1 and R2 groups each represents an alkyl group having one to three carbon atoms; R3 group represents a hydrogen or an alkyl group having one to three carbon atoms; and R1 and R3 together may form a ring).
[9]
   The method of [8], wherein the compound of formula (I) is an imine derivative represented by formula (III) below and the compound of formula (II) is an amine derivative represented by formula (IV): 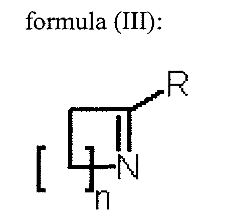 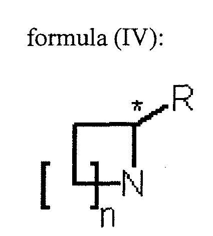 (wherein R group represents an alkyl group having one to three carbon atoms; and n represents an integer of 1 to 4).
[10]
   The method of [9] for producing an optically active R-isomer amine derivative, wherein the compound represented by formula (III) is 2-methyl-1-pyrroline and the compound represented by formula (IV) is 2-methylpyrrolidine.
[11]
   The method of any one of [8] to [10] for producing an optically active R-isomer amine derivative, wherein the microorganism belongs to the genus *Streptomyces.*
[12]
   The method of [11] for producing an optically active R-isomer amine derivative, wherein the microorganism belonging to the genus *Streptomyces* is any one selected from the group consisting of:
   *Streptomyces* sp. NITE BP-594;
   *Streptomyces* sp. NITE BP-595;
   *Streptomyces* sp. NITE BP-596; and
   *Streptomyces* sp. NITE BP-597.
[13]
   The method of [11] for producing an optically active R-isomer amine derivative, wherein the microorganism belonging to the genus *Streptomyces* is any one selected from the group consisting of:
   *Streptomyces griseorubiginosus;*
   *Streptomyces microflavus;* and
   *Streptomyces alboviridis.*
[14]
   The method of [13] for producing an optically active R-isomer amine derivative, wherein *Streptomyces griseorubiginosus, Streptomyces microflavus,* and *Streptomyces alboviridis* are the following microbial strains:
   *Streptomyces griseorubiginosus* NBRC 13047;
   *Streptomyces microflavus* NBRC 13062; and
   *Streptomyces alboviridis* NBRC 13013, respectively.
[15]
   A method for producing an optically active R-isomer amine derivative, which comprises the steps of:
   contacting an imine derivative represented by formula (I) with at least one enzymatically active material selected from the group consisting of:
   the imine reductase of any one of [1] to [7]; a microorganism that produces the imine reductase; and a processed product thereof; and
   collecting the optically active R-isomer amine derivative represented by formula (II): 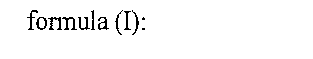 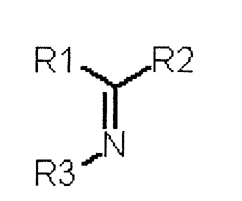 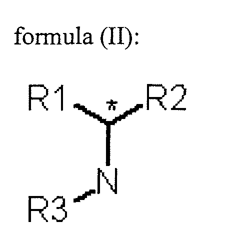 (wherein R1 and R2 groups each represents an alkyl group having one to three carbon atoms; R3 group represents a hydrogen or an alkyl group having one to three carbon atoms; and R1 and R3 together may form a ring).
[16]
   The method of [15], wherein the compound represented by formula (I) is an imine derivative represented by formula (III) below and the compound of formula (II) is an amine derivative represented by formula (IV): 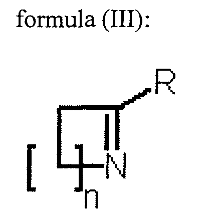 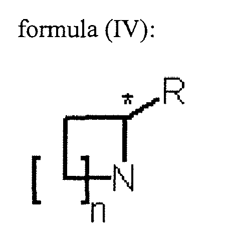 (wherein R group represents an alkyl group having one to three carbon atoms; and n represents an integer of 1 to 4).
[17]
   A polynucleotide encoding an imine reductase that has the physicochemical properties of (1) and (2) of [1], which is any one of the following polynucleotides:
   (a) a polynucleotide comprising the nucleotide sequence of SEQ ID NO: 5;
   (b) a polynucleotide encoding a protein comprising the amino acid sequence of SEQ ID NO: 6;
   (c) a polynucleotide encoding a protein comprising an amino acid sequence with a substitution, deletion, insertion, and/or addition of one or more amino acids in the amino acid sequence of SEQ ID NO: 6;
   (d) a polynucleotide that hybridizes under a stringent condition to a DNA comprising the nucleotide sequence of SEQ ID NO: 5; and
   (e) a polynucleotide encoding an amino acid sequence with 80% or higher sequence identity to the amino acid sequence of SEQ ID NO: 6.
[18]
   A polynucleotide encoding an imine reductase that has the physicochemical properties of (1) and (2) of [1] and the function of generating (R)-2-methylpyrrolidine with at least 80%ee or higher optical purity, and is any one of the following polynucleotides:
   (a) a polynucleotide comprising the nucleotide sequence of SEQ ID NO: 16;
   (b) a polynucleotide encoding a protein comprising the amino acid sequence of SEQ ID NO: 17;
   (c) a polynucleotide encoding a protein comprising an amino acid sequence with a substitution, deletion, insertion, and/or addition of one or more amino acids in the amino acid sequence of SEQ ID NO: 17;
   (d) a polynucleotide that hybridizes under a stringent condition to a DNA comprising the nucleotide sequence of SEQ ID NO: 16; and
   (e) a polynucleotide encoding an amino acid sequence with 80% or higher sequence identity to the amino acid sequence of SEQ ID NO: 17.
[19]
   A recombinant vector inserted with the polynucleotide of [17] or [18].
[20]
   The recombinant vector of [19], which is inserted additionally with a polynucleotide encoding a dehydrogenase that is capable of catalyzing a redox reaction using β-nicotinamide adenine dinucleotide phosphate as a coenzyme.
[21]
   The vector of [20], wherein the dehydrogenase is a glucose dehydrogenase.
[22]
   The recombinant vector of [21], wherein the glucose dehydrogenase is derived from *Bacillus subtilis.*
[23]
   A transformant that maintains the polynucleotide of [17] or [18] or the vector of [19] in an expressible manner.
[24]
   A method for producing a protein encoded by the polynucleotide of [17] or [18], which comprises the step of culturing the transformant of [23] and collecting an expression product.

### [Effects of the Invention]

The present invention provides methods for efficiently producing optically active amine (R) isomers by stereoselectively reducing imines using the reducing ability of microorganisms. The present invention also provides imine reductases that catalyze the reaction. In addition, the present invention provides recombinant bacteria expressing the enzymes at high levels by isolating DNAs encoding the above-described imine reductases. The methods of the present invention which use the high stereoselectivity of a microorganism or enzyme are industrially advantageous because optically active amines can be synthesized using as a substrate an imine derivative that can be synthesized at low cost. The present invention is particularly useful in producing (R)-2-methylpyrrolidine. (R)-2-Methylpyrrolidine is a useful compound as an intermediate in pharmaceutical synthesis. The present invention enables simple, efficient production of the compound by using the activity of microorganisms or enzymes

### Brief Description of the Drawings

Fig. 1 is a diagram showing a molecular phylogenetic tree constructed based on the nucleotide sequence of 16S rDNA (SEQ ID NO: 1) of *Streptomyces* sp. GF3587.
Fig. 2 is a diagram showing a molecular phylogenetic tree constructed based on the nucleotide sequence of 16S rDNA (SEQ ID NO: 2) of *Streptomyces* sp. GF3501.
Fig. 3 is a diagram showing a molecular phylogenetic tree constructed based on the nucleotide sequence of 16S rDNA (SEQ ID NO: 3) of *Streptomyces* sp. GF3585.
Fig. 4 is a diagram showing a molecular phylogenetic tree constructed based on the nucleotide sequence of 16S rDNA (SEQ ID NO: 4) of *Streptomyces* sp. GF4415.
Fig. 5 is a graph showing a result of 2-methyl-1-pyrroline (2-MPN) reduction by *Streptomyces* sp. GF3587. The vertical axis indicates the amount (mM) of (R)-2-methylpyrrolidine (2-MP) produced, while the horizontal axis indicates reaction time (hour).
Fig. 6 is a graph showing a result of 2-methyl-1-pyrroline (2-MPN) reduction by *Streptomyces* sp. GF3587. The vertical axis indicates the amount (mM) of (R)-2-methylpyrrolidine (2-MP) produced, while the horizontal axis indicates reaction time (hour).
Fig. 7 is a graph showing a result of assessing the optimal temperature of imine reductase derived from *Streptomyces* sp. GF3587. The vertical axis indicates the relative activity (%) when the maximal activity (12.3 U/ml) at 50°C is taken as 100, while the horizontal axis indicates the temperature (°C) of enzyme reaction mixture.
Fig. 8 is a graph showing a result of assessing the thermal stability of imine reductase derived from *Streptomyces* sp. GF3587. The vertical axis indicates the relative activity (%) when the maximal residual activity (6.65 U/ml) at 20 to 25°C is taken as 100, while the horizontal axis indicates the enzyme-treating temperature (°C).
Fig. 9 is a graph showing a result of assessing the optimal pH of imine reductase derived from *Streptomyces* sp. GF3587. The vertical axis indicates the relative activity (%) when the maximal activity (6.82 U/ml) at pH 7.0 is taken as 100, while the horizontal axis indicates the pH of enzyme reaction mixture.
Fig. 10 is a graph showing a result of assessing the pH stability of imine reductase derived from *Streptomyces* sp. GF3587. The vertical axis indicates the relative activity (%) when the maximal residual activity (3.67 U/ml) at pH 7.5 is taken as 100, while the horizontal axis indicates the enzyme-treating pH.
Fix. 11 is a diagram showing the construction process of plasmid (pSUSRIR1) inserted with the imine reductase gene derived from *Streptomyces* sp. GF3587.
Fig. 12 is a diagram showing the construction process of plasmid (pSGSRIR1) inserted with the *Streptomyces* sp. GF3587-derived imine reductase gene and the *Bacillus*-derived glucose dehydrogenase gene.
Fig. 13 is a diagram showing the construction process of plasmid (pSG-SKI1) inserted with Q1EQE0 and the *Bacillus*-derived glucose dehydrogenase gene.

### Mode for Carrying Out the Invention

The present invention relates to methods for producing optically active R-isomer amine derivatives, which comprise the steps of:
contacting an imine derivative represented by formula (I) with at least an enzymatically active material selected from the group consisting of a culture of a microorganism having the ability of stereoselectively reducing the compound, the microbial cells, and processed products of the cells;
and collecting the optically active R-isomer amine derivative represented by formula (II). 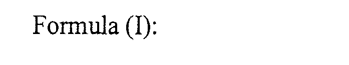 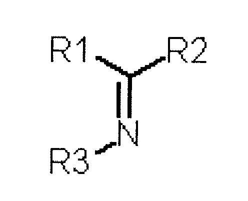 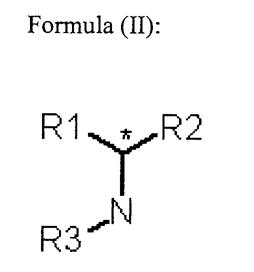
(wherein R1 and R2 groups each represents an alkyl group having one to three carbon atoms; R3 group represents a hydrogen or an alkyl group having one to three carbon atoms; and R1 and R3 may form a ring)

In the present invention, cyclic imine compounds are preferred compounds of formula (I). In the present invention, the cyclic imine compounds include, for example, imine derivatives represented by formula (III) below. 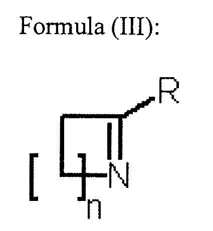 (wherein R group represents an alkyl group having one to three carbon atoms; and n represents an integer of 1 to 4)

The compound represented by formula (IV) as an optically active amine derivative can be produced by using as a substrate a compound represented by formula (III) above. The compound is a preferred optically active amine derivative that can be produced according to the present invention. 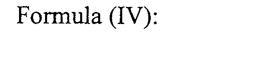 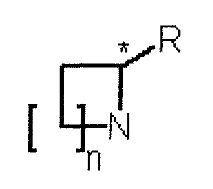 (wherein R group represents an alkyl group having one to three carbon atoms; and n represents an integer of 1 to 4)

In preferred compounds of the present invention, for example, R is a methyl group, ethyl group, or propyl group, and n is 2 or 3 in formula (III) above. More specifically, preferred imine derivatives of the present invention include:
2-methyl-1-pyrroline;
2-methyl-1-piperideine;
2-ethyl-1-pyrroline; and
2-ethyl-1-piperideine.

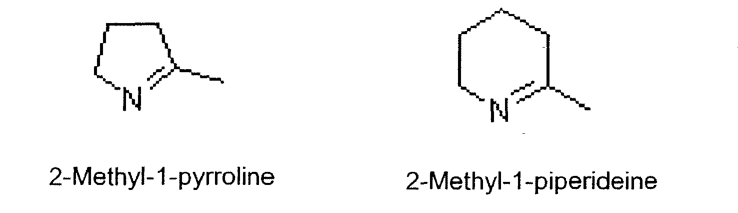

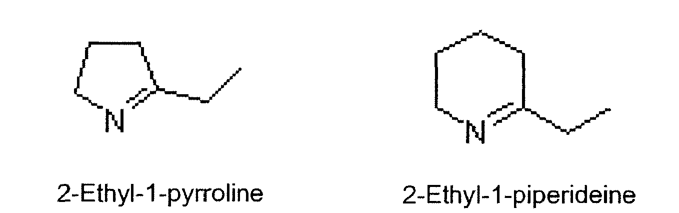

The optically active amine derivatives shown below can be produced by using the above-described compounds as substrates.
2-methyl-1-pyrroline → (R)-2-methylpyrrolidine
2-methyl-1-piperideine → (R)-2-methylpiperidine
2-ethyl-1-pyrroline → (R)-2-ethylpyrrolidine
2-ethyl-1-piperideine → (R)-2-ethylpiperidine

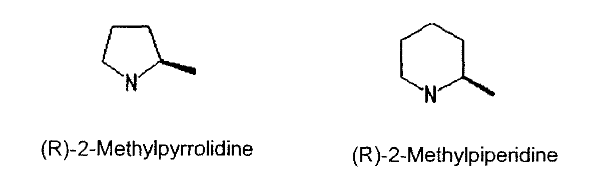

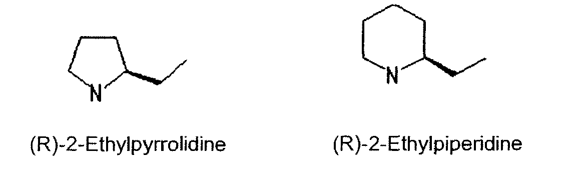

Herein, "optically active amine derivatives" refers to amine derivatives in which a certain type of optical isomer is more abundant than the other optical isomer. In the present invention, preferred optically active amine derivatives have, for example 60%, typically 70% or higher, preferably 80% or higher, and still more preferably 90% or higher optical purity (enantiomeric excess (ee)). In general, the "optical isomer" of the present invention is sometimes also referred to as an "enantiomer".

Herein, the ability to stereoselectively reduce an imine derivative refers to the ability to generate an R-isomer amine derivative represented by formula (II) or (IV) using as a substrate an imine derivative represented by formula (I) or desirably formula (III) above. The microorganism of the present invention may be any microorganism, as long as it can generate an R-isomer amine derivative. Such microorganisms of the present invention can be obtained by testing whether a microorganism has the ability to generate an R-isomer amine derivative when it acts on an imine derivative.

For example, a test microorganism is cultured in a medium containing an imine derivative represented by compound (I), desirably compound (III), and then the culture medium is assayed to determine the optical purity of the amine derivative generated. As a result, if the generation of an optically active (R)-amine derivative can be demonstrated, the microorganism can be used in the present invention.

Alternatively, a test microorganism is grown in advance in an appropriate medium, and the microbial cells are harvested and suspended in an appropriate buffer. Then, the cells are contacted and reacted with an imine derivative represented by compound (I), desirably compound (III). The optical purity of the amine derivative generated in the reaction buffer is determined. When the generation of an optically active (R)-amine derivative is demonstrated, the microorganism can be used in the present invention. In this case, when compound (I), preferably compound (III) is added to the culture medium during the culture of the test microorganism, induction of the enzyme for reducing the compound is expected. Furthermore, a more effective accumulation of the product can be expected when an energy source for the reduction is added during the reaction. Such energy sources for the reduction typically include glucose, sucrose, alcohols, and organic acids.

The ability of a microorganism to reduce 2-methyl-1-pyrroline can be assessed, for example, by the following procedure. A microorganism is cultured in an appropriate medium containing, if needed, 2-methyl-1-pyrroline. The resulting microbial cells are suspended in a reaction mixture containing 25 mM potassium phosphate buffer (pH 7.0), 10 mM 2-methyl-1-pyrroline, and glucose as needed. The suspension is shaken at 25°C for 24 hours, and then the product is analyzed by TLC or HPLC.

TLC conditions include, for example, a method that uses Silica gel 60 F254 plate, and develops using the developing solvent 1-butanol/acetic acid/water = 2/1/1, followed by color reaction using ninhydrin.

The optical purity of the generated amine derivative can be determined, for example, by the following method.

The generated amine is derivatized with GITC by adding the following components to 50 µl of test sample and incubating the mixture at 40°C for one hour, and is then isolated and quantified by HPLC.

50 µl of 2 mg/ml triethylamine and 100 µl of 8 mg/ml
2,3,4,6-tetra-O-acetyl-β-D-glucopyranosyl isothiocyanate(GITC)

HPLC can be performed, for example, under the following conditions.
- HPLC column: Wakosil II 5C18 (4.6 mm x 150 mm) from Wako Pure Chemical Industries
- elution buffer: 10 mM phosphate buffer (pH 2.5):methanol = 55:45
- flow rate: 1 ml/min
- detection: UV absorbance at 254 nm

Under the conditions described above, the (R)-2-methylpyrrolidine derivative is eluted at 26.3 minutes, while the (S)-2-methylpyrrolidine derivative is eluted at 24.9 minutes.

Needless to say, it is advantageous to use microorganisms with high stereoselectivity to obtain high optical purity products. Specifically, microorganisms which can synthesize optically active R-isomer amine derivatives with an optical purity of, for example 60%, typically 70% or higher, preferably 80% or higher, and more preferably 90% or higher, can be used.

The microorganisms that generate R-isomer amine derivatives include, for example, those that belong to the genus *Streptomyces* and have the ability to generate (R)-2-methylpyrrolidine from 2-methyl-1-pyrroline. For example, the microorganisms listed below can be used to generate R-isomer amine derivatives.
*Streptomyces* sp. GF3587;
*Streptomyces* sp. GF3501;
*Streptomyces* sp. GF3585; and
*Streptomyces* sp. GF4415.

These microorganisms are deposited in the following depositary institution.
(1) Name of depositary institution: Incorporated Administrative Agency National Institute of Technology and Evaluation
(2) Address: Postal code: 292-0818 2-5-8 Kazusakamatari, Kisarazu-shi, Chiba, Japan
(3) Identification display and accession number:
   *Streptomyces* sp. GF3587
      Accession number: NITE P-594 (Date of deposition: June 24, 2008)
   *Streptomyces* sp. GF3585
      Accession number: NITE P-596 (Date of deposition: June 24, 2008)
   *Streptomyces* sp. GF3501
      Accession number: NITE P-595 (Date of deposition: June 24, 2008) *Streptomyces* sp. GF4415
      Accession number: NITE P-597 (Date of deposition: June 24, 2008)

Furthermore, these microorganisms have been transferred to the following international depositary authority based on the Budapest Treaty.
(1) International depositary authority: Incorporated Administrative Agency National Institute of Technology and Evaluation,
   NITE Patent Microorganisms Depositary (NPMD)
(2) Address: Postal code: 292-0818 2-5-8 Kazusakamatari, Kisarazu-shi, Chiba, Japan
(3) Identification display and accession number:
   *Streptomyces* sp. GF3587
      Accession number: NITE BP-594 (Date of transfer: June 29, 2009)
   *Streptomyces* sp. GF3585
      Accession number: NITE BP-596 (Date of transfer: June 29, 2009)
   *Streptomyces* sp. GF3501
      Accession number: NITE BP-595 (Date of transfer: June 29, 2009)
   *Streptomyces* sp. GF4415
      Accession number: NITE BP-597 (Date of transfer: June 29, 2009)

In the present invention, microorganisms having the ability to stereoselectively reduce an imine derivative represented by formula (I) or (III) described above are not limited to the above deposited bacterial strains. Such microorganisms can be obtained from various microbiological resources. The microbiological resources include those isolated from natural environment and those stored in depositary institutes of microbiology. For example, the microorganisms of the genus *Streptomyces* listed below are preferred microorganisms having the ability to stereoselectively reduce imine derivatives represented by formula (I) or (III) described above.
*Streptomyces griseorubiginosus*
*Streptomyces microflavus*
*Streptomyces alboviridis*

Thus, for example, the above-described selection methods can be used to select microorganisms having an activity of interest from microorganisms of the genus *Streptomyces* deposited in depositary institutes of microbiology. Such microorganisms deposited are available, for example, from the following depositary institutes.
NITE Biological Resource Center (NBRC)

The Institute of Physical and Chemical Research (JCM)
NODAI Culture Collection Center (IAM)
American Type Culture Collection (ATCC)
Deutsche Sammlung von Mikroorganismen (DSM)

Alternatively, microorganisms of the present invention can be selected based on nucleotide sequence information on ribosomal DNA (rDNA). It is well known that the nucleotide sequence information on rDNA is generally useful as an indicator for the genetic distance between organisms. In particular, DNAs encoding 16S rRNA (hereinafter abbreviated as 16S rDNA) have been used as a tool for determining the genetic identity or homology between microorganisms. 16S rRNA is present in all organisms. Although the structure is different between organisms, the sequence features are conserved to a degree that allows sequence alignment. The frequency of horizontal transmission of 16S rRNA between organisms is low, and thus 16S rRNA has been commonly used to classify organisms.

Specifically, small subunit ribosomal RNA (SSU rRNA) is a nucleic acid molecule that constitutes a ribosome which is a site of protein synthesis. The lengths of the nucleotide sequences are slightly different, but the origin is considered to be the same from bacteria to human beings. Thus, the nucleotide sequence of rRNA has been highly conserved during evolution and has been most frequently used in phylogenetic analysis of organisms (Microbiol. Rev., 58,1-9 (1994)). Prokaryotic SSU rRNA has about 1,500 nucleotides. A systematic taxonomy based on the nucleotide sequence of 16S rRNA is generally used to identify and classify prokaryotes (ASM News, 65, 752-757 (1999); Seibutsu Kogaku Jikkensho (Experimental manual of bioengineering) p. 113, Ed. The Society of Biotechonogy, Japan, Baifukan).

The current classification system of actinomycetes is constituted based on the homology of the nucleotide sequence of 16S rDNA (Stackebrandt, et al. (1997) Int. J. Syst. Bacteriol., 47, 479-491). In the major bacterial systematic taxonomy, bacterial strains are identified not only at the genus level but also at the species level based on the identity of nucleotide sequence of 16S rDNA ("Housenkin no Bunrui to Doutei (Identification and classification of actinomycetes)" (The Society ofActinomycetes Japan) p. 19).

16S rDNAs of bacterial strains identified by the present inventors were found to comprise the nucleotide sequences of SEQ ID NOs: 1 to 4. Nucleotide sequence information databases can be searched with this nucleotide sequence information to find bacterial strains having a highly homologous nucleotide sequence in their 16S rDNAs from microorganisms whose 16S rDNA has already been determined. Public database service providers, for example, DNA Data Bank of Japan (DDBJ), provide data search services that target the nucleotide sequence information on 16S rRNA (Prokaryotes). Of the microorganisms revealed by this method, microorganisms belonging to the genus *Streptomyces* are selected in particular and can be used in the present invention. If needed, microorganisms selected by the above-described method may be assessed in advance for the activity on imine derivatives.

Indeed, the present inventors experimentally demonstrated that microorganisms selected by the above-described method had the desired catalytic activity. Specifically, the microorganisms of the genus *Streptomyces* listed below, whose 16S rDNA sequences have 99.9% identity to that of GF3587 strain, were demonstrated to generate high optical purity (R)-2-methylpyrrolidine by reducing 2-methyl-1-pyrroline in an optically specific manner.
*Streptomyces griseorubiginosus* NBRC 13047
*Streptomyces microflavus* NBRC 13062
*Streptomyces alboviridis* NBRC 13013

These microorganisms can be cultured according to methods known in the field of zymology. Culture medium may be a synthetic or natural medium as long as it contains appropriate amounts of carbon source, nitrogen source, inorganic material, and other nutrients. The medium may be a liquid or solid medium.

Specifically, by considering the assimilation in a microorganism to be used, one, two, or more carbon sources are appropriately selected from those commonly used as listed below.
Sugars: glucose, fructose, maltose, sucrose, lactose, etc.;
starch;
starch hydrolysate;
molasses;
alcohols: glycerol, methanol, ethanol, etc.;
sugar alcohols;
organic acids: acetic acid, gluconic acid, pyruvic acid, α-ketoglutaric acid, citric acid, etc.;
lipids: palm oil, soybean oil, olive oil, etc.; and
hydrocarbons: normal paraffin, etc.

By considering the assimilation in a microorganism to be used, one, two, or more nitrogen sources are appropriately selected from those commonly used as listed below.

Organic nitrogen compounds: for example, meat extract, peptone, yeast extract, malt extract, soybean hydrolysate, milk casein, casamino acid, molasses, amino acids such as asparagine, glutamine, and glutamic acid, corn steep liquor, NZ amines, etc.

Inorganic nitrogen compounds: ammonia, ammonium nitrate, ammonium sulfate, etc.
urea, nitrate salt, etc.

Furthermore, it is possible to add as inorganic salts a small amount of a salt of magnesium, manganese, potassium, calcium, sodium, copper, zinc, or such, and trace nutrients such as various vitamins.

In addition, antifoaming agents such as vegetable oils, detergents, and silicon may be added to the culture medium, if required. Moreover, an inducing substance may be used to enhance the ability of a microorganism to stereoselectively reduce imine derivatives. Imine derivatives can be used as an inducing substance depending on the microorganism used.

Alternatively, preferable culture conditions can be used to induce the production of an enzyme of interest without using an inducing substance. For example, the present inventors successfully induced the imine reductase in the genus *Streptomyces* by using the medium described below, which was optimized for the induction of the imine reductase. Specifically, the present invention provides methods for producing an imine reductase, which comprise the steps of culturing a microorganism of the genus *Streptomyces* in a culture medium having the composition described below, and collecting the imine reductase from the culture.
4 g of NZ amine
20 g of malt extract
8 g of glucose
5 mg of FeSO₄·7H₂O

The ingredients described above are dissolved in tap water to 1 L and pH is adjusted to 7.3.

By culturing, for example, microorganisms described below in a culture medium having the above-described composition, the production of an imine reductase of the present invention can be induced without adding any inducing agents such as imine derivatives.
*Streptomyces griseorubiginosus*
*Streptomyces microflavus*
*Streptomyces alboviridis*

These bacteria can be cultured by conventional culture methods using liquid medium having the above-described components. For example, it is possible to use the following culture methods.
shaking culture
aeration-agitation culture
continuous culture
feeding culture, fed batch culture

Appropriate culture conditions can be selected depending on the type of microorganisms, type of culture, and culture methods. The conditions are not particularly limited as long as they allow the bacterial strain to be used to grow and to have the ability to stereoselectively reduce imine derivatives. General culture conditions are described below.
pH at the start of culture is adjusted to 4 to 10, preferably 6 to 8.

The culture temperature is 15 to 70°C, preferably 20 to 40°C.

The culture period is not particularly limited, as long as it allows expansion of microbial cells having the ability to reduce imine derivatives. Typically, the culture period is 1 to 14 days, preferably 1 to 7 days.

Herein, the "processed materials of microbial cells" refers to materials from treating the microbial cells under conditions that allow them to retain the enzymatic activity of interest. Retainment of enzymatic activity means that the retained enzymatic activity is generally 20% or more as compared to that of viable microbial cells, typically 30% or more, preferably 50% or more, and more preferably 70% or more. The enzymatic activity can be quantitatively compared between viable microbial cells and the processed material thereof by assaying the activity of viable microbial cells and that of the processed material prepared from the same amount of viable microbial cells. In some cases, the enzymatic activity of the processed materials of the present invention may be higher than that of viable microbial cells.

Methods for preparing such processed materials include lyophilization, dehydration drying with an organic solvent, autolysis of microbial cells, extraction of enzymatically active fractions, and sonication. Such processing methods yield, for example, lyophilized microbial cells, acetone-dried microbial cells, microbial cell autolysate, microbial cell extract, crushed product of microbial cells, and sonication product of microbial cells.

These processing methods may be used alone or in combination. For example, after culturing a microorganism in an appropriate culture medium, the microbial cells can be crushed and then centrifuged to prepare cell-free extract. The microbial cells can be crushed mechanically, or by sonication, high pressure treatment, enzymatic digestion, autolysis, or such. Cell-free extracts are included in the processed materials of microbial cells of the present invention.

Furthermore, in the present invention, enzymes that catalyze the reaction can be obtained by partially or completely purifying the microbial cells. More specifically, the present invention relates to imine reductases that can be purified from microbial cells of microorganisms provided by the present invention which have an imine reductase activity. The imine reductases of the present invention have the following physicochemical properties:
(1) activity: to produce (R)-2-methylpyrrolidine by reducing 2-methyl-1-pyrroline in a reduced nicotinamide adenine dinucleotide phosphate-dependent manner; and
(2) coenzyme dependency: use NADPH as a coenzyme.
In a preferred embodiment, the imine reductases discovered by the present inventors have, in addition to the properties described above in (1) and (2), the physicochemical properties described below. Such imine reductases can be prepared, for example, from *Streptomyces* sp. GF3587.
(3) molecular weight: 65,000 to 70,000 by gel filtration; and approximately 32,000 by SDS-PAGE.
(4) optimal pH: pH 6.5 (at pH 6.0 to 7.5, 80% or more of the maximal activity)
(5) optimal temperature: 50°C (at 40 to 55°C, 80% or more of the maximal activity)
(6) pH stability: the stability is 80% or higher at pH 6.5 to 8.5 (residual activity after 30 minutes of treatment at 30°C)
(7) thermal stability: the stability is 80% or higher up to 35°C (residual activity after 30 minutes of treatment at each temperature)
(8) substrate specificity: the level of activity of reducing each of the compounds indicated below, when the activity of reducing 2-methyl-1-pyrroline is taken as 100.

| | |
|---|---|
| 2-methyl-1-pyrroline: | 100% |
| 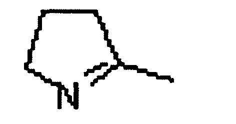 | |
| N-benzylidenebenzylamine: | 30% |
| 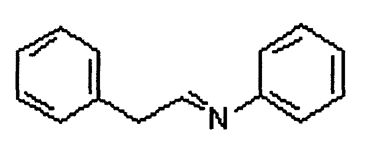 | |
| N-(E)-N-(1-phenylethylidene)aniline: | 29% |
| 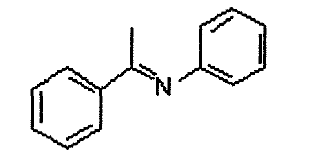 | |
| dihydrofolic acid: | 7.6% |
| 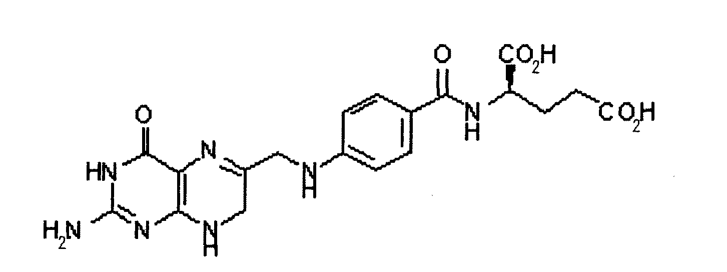 | |

The imine reductases of the present invention can be purified from the above-described microorganisms, cell-free extract thereof, or such. More specifically, substantially pure imine reductases can be obtained from cell-free extract by using appropriate combinations of the following purification steps:
fractionation based on solubility using ammonium sulfate, acetone, or such;
ion exchange chromatography;
hydrophobic chromatography;
affinity chromatography using 2',5'-ADP Sepharose, blue or red Sepharose, or such; and
gel filtration.

After harvesting microbial cells, an imine reductase of the present invention can be isolated as a substantially pure enzyme, for example, by the following purification steps:
crushing microbial cell by sonication, followed by preparation of cell-free extract;
isolation of protein fraction by ammonium sulfate fractionation;
ion exchange chromatography using DEAE-Sephacel;
hydrophobic chromatography using Phenylsepharose; and
isolation of the fraction having the imine reductase activity by 2',5'-ADP Sepharose affinity chromatography.

Herein, "substantially pure" means that an enzyme does not substantially contain biological macromolecular compounds or chemical substances other than itself. Such biological macromolecular compounds other than the enzyme include, for example, proteins, sugars, lipids, and nucleic acids, which are derived from microbial cells and culture medium.

The purity of a substantially pure enzyme of the present invention is at least 75% or higher, typically 80% or higher, preferably 85% or higher, more preferably 90% or higher, still more preferably 95% or higher, or 99% or higher. Methods for determining enzyme purity are known. Known methods for determining protein purity include, for example, various chromatographic methods and polyacrylamide gel electrophoresis.

The present invention also provides isolated imine reductases having the physicochemical properties described above in (1) and (2). Herein, the isolated imine reductase means that the reductase is separated from the natural environment where it originally occurs. Thus, imine reductases separated from microbial cells are included in the isolated imine reductases of the present invention.

A substantially pure imine reductase of the present invention or isolated imine reductase of the present invention may be formulated into an enzyme composition after purification or isolation. For example, an enzyme composition comprising an imine reductase of the present invention can be prepared by formulating a purified or isolated imine reductase with appropriate carriers. Such carriers include inactive proteins such as albumin, sugars such as sucrose, and sugar alcohols. The enzyme composition may be liquid or in a lyophilized form. Enzyme compositions prepared by lyophilizing aqueous solutions containing the enzyme and carriers are a preferred composition of the present invention.

The activity of an imine reductase of the present invention can be quantitatively determined by the method described below. More specifically, the reductase is incubated at 30°C in a reaction solution (1 mL) having the composition described below. The reaction mixture is assayed to determine the decrease in absorbance at 340 nm due to the decrease of NADPH.
10 mM 2-methyl-1-pyrroline;
100 mM potassium phosphate buffer (pH 7.0);
0.1 mM NADPH; and
enzyme.

The amount of enzyme that catalyzes the decrease of 1 µmol of NADPH per minute under the above-described conditions is defined as 1 U.

The present invention relates to proteins comprising the amino acid sequence of SEQ ID NO: 6 or 17. The present invention also includes homologues of a protein comprising the amino acid sequence of SEQ ID NO: 6 or 17. A homologue of an imine reductase of the present invention refers to a protein having an amino acid sequence with a deletion, substitution, insertion, and/or addition of one or more amino acids in the amino acid sequence of SEQ ID NO: 6 or 17, which is functionally equivalent to a protein comprising the amino acid sequence of SEQ ID NO: 6 or 17. Mutations of, for example, 100 amino acid residues or less, typically 50 or less, preferably 30 or less, more preferably 15 or less, and still more preferably 10 or less, or 5 or less are acceptable in the amino acid sequence of SEQ ID NO: 6 or 17.

The present invention relates to polynucleotides encoding an imine reductase and homologues thereof. The polynucleotides of the present invention include natural polynucleotides such as DNAs and RNAs, and artificial molecules containing artificial nucleotide derivatives. The polynucleotides of the present invention also include chimeric DNA-RNA molecules. A polynucleotide encoding an imine reductase of the present invention comprises, for example, the nucleotide sequence of SEQ ID NO: 5. The nucleotide sequence of SEQ ID NO: 5 encodes a protein comprising the amino acid sequence of SEQ ID NO: 6. In a preferred embodiment, an imine reductase of the present invention is a protein comprising the amino acid sequence.

A homologue of an imine reductase encoding an imine reductase of the present invention includes an amino acid sequence with a deletion, substitution, insertion, and/or addition of one or more amino acids in the amino acid sequence of SEQ ID NO: 6, and includes a polynucleotide encoding a protein having the physicochemical properties described above in (1) and (2). Those skilled in the art can obtain a polynucleotide homologue by appropriately introducing substitutional, deletional, insertional, and/or additional mutations into the polynucleotide of SEQ ID NO: 5 by site-directed mutagenesis (Nucleic Acid Res. 10, pp. 6487 (1982); Methods in Enzymol. 100, pp. 448 (1983); Molecular Cloning 2nd Edt., Cold Spring Harbor Laboratory Press (1989); PCR A Practical Approach IRL Press pp. 200 (1991)) or the like.

Polynucleotide homologues of the present invention also include polynucleotides that are capable of hybridizing under stringent conditions with a polynucleotide comprising the nucleotide sequence of SEQ ID NO: 5, and which encode a protein having the physicochemical properties (1) and (2) described above. The term "polynucleotides capable of hybridizing under stringent conditions" refers to a polynucleotide that hybridizes to one or more DNA probes that have an arbitrary sequence of at least 20, preferably at least 30, for example, 40, 60, or 100 constitutive nucleotides of SEQ ID NO: 5, using the ECL direct nucleic acid labeling and detection system (GE Healthcare) under conditions described in the manual (washing at 42°C in a primary wash buffer containing 0.5x SSC).

The polynucleotide homologue of the present invention includes a polynucleotide encoding a protein having an identity of at least 70%, preferably at least 80%, much more preferably 90% or higher to the amino acid sequence of SEQ ID NO: 6. Protein identity searches can be performed, for example, against protein amino acid sequence databases such as SWISS-PROT, PIR, and DAD; DNA sequence databases such as DDBJ, EMBL, and GenBank; and databases of amino acid sequences predicted based on DNA sequences by using programs such as BLAST and FASTA *via* the Internet.

Identity search of the amino acid sequence of SEQ ID NO: 6 was performed in DAD using the BLAST program. The result showed that of known proteins, proteins exhibiting high identity were Q1EQE0 (51%) and A3KHV9 (42%) produced by *Streptomyces kanamyceticus* and *Streptomyces ambofaciens,* respectively. Herein, 70% or higher identity refers to values calculated, for example, using a program based on the Lipman-Pearson method (Science, 227, 1435-1441 (1985)).

To demonstrate whether the predicted proteins have the imine reductase activity of the present invention, primers were synthesized based on the DNA sequences registered in DDBJ and the putative ORF portions were cloned by PCR from the genomic DNAs of *Streptomyces kanamyceticus* and *Streptomyces ambofaciens.* Each ORF was inserted into an expression vector, and *E. coli* was transformed with the resulting vectors. Each transformant obtained was cultured to express the protein. As a result, Q1EQE0 exhibited the imine-reducing activity. In addition, Q1EQEO produced high optical purity (R)-2-methylpyrrolidine by reducing 2-methyl-1-pyrroline *via* imine reduction reaction. Specifically, proteins comprising the amino acid sequence of SEQ ID NO: 17 are a preferred embodiment of the imine reductase homologue of the present invention.

The present invention also relates to a protein comprising the amino acid sequence of SEQ ID NO: 6. The present invention also includes homologues of the protein comprising the amino acid sequence of SEQ ID NO: 6.

The "imine reductase homologues of the present invention" refers to proteins that are functionally equivalent to a protein comprising the amino acid sequence of SEQ ID NO: 6 and comprise the amino acid sequence with a deletion, substitution, insertion, and/or addition of one or more amino acids in the amino acid sequence of SEQ ID NO: 6. Herein, the phrase "functionally equivalent to the protein comprising the amino acid sequence of SEQ ID NO: 6" means that the protein has the physicochemical properties (1) and (2) described above. Those skilled in the art can obtain a polynucleotide encoding a homologue of the imine reductase by appropriately introducing substitutional, deletional, insertional, and/or additional mutations into the DNA of SEQ ID NO: 5 by site-directed mutagenesis (Nucleic Acid Res. 10, pp. 6487 (1982); Methods in Enzymol. 100, pp. 448 (1983); Molecular Cloning 2nd Edt., Cold Spring Harbor Laboratory Press (1989); PCR A Practical Approach IRL Press pp. 200 (1991)) or the like. A homologue of the imine reductase of SEQ ID NO: 6 can be obtained by introducing and expressing a polynucleotide encoding the imine reductase homologue within a host.

Furthermore, the imine reductase homologue of the present invention refers to a protein having an identity of at least 70%, preferably at least 80%, more preferably 90%, much more preferably 90% or higher to the amino acid sequence of SEQ ID NO: 6. Protein identity searches can be performed against protein amino acid sequence databases, such as SWISS-PROT, PIR, and DAD; DNA sequence databases, such as DDBJ, EMBL, and GenBank; and databases of amino acid sequences predicted based on DNA sequences by using programs such as BLAST and FASTA *via* the Internet.

Polynucleotides encoding the imine reductases of the present invention can be isolated, for example, by the methods described below.

PCR can be performed to obtain the DNAs of the present invention, using PCR primers designed based on the nucleotide sequence of SEQ ID NO: 5, and either chromosomal DNA from a strain producing the enzyme, or a cDNA library, as a template.

Additionally, the polynucleotides of the present invention can be obtained by digesting chromosomal DNA from a strain producing the enzyme with restriction enzymes, and then inserting these fragments into a phage, plasmid, or the like; and transforming *E. coli* with the DNA construct. The resulting library, or a cDNA library, can be screened by colony hybridization, plaque hybridization, or similar methods, using the DNA fragment obtained as described above as a probe.

Alternatively, the polynucleotides of the present invention can be obtained by analyzing the nucleotide sequence of the DNA fragment yielded by PCR, and then designing PCR primers for sequence extension based on the sequence obtained. Next, chromosomal DNA of the enzyme-producing strain is digested with appropriate restriction enzymes, and then subjected to self-circle formation to serve as a template for inverse PCR (Genetics 120, 621-623 (1988)), or alternatively by RACE (Rapid Amplification of cDNA End; "Experimental manual for PCR" p.25-33, HBJ Press), and such.

The polynucleotides of the present invention include synthetic DNAs in addition to genomic DNAs and cDNAs cloned by the method described above.

The present invention also provides expression vectors for the imine reductase which are constructed by inserting a polynucleotide encoding the imine reductase of the present invention isolated by the method described above into a conventional expression vector.

The imine reductase of the present invention can be obtained from recombinants by culturing the recombinants transformed with the expression vector.

There is no limitation on the type of microorganism to be transformed for the expression of the imine reductase of the present invention, as long as it is capable of being transformed with a recombinant vector that contains a polynucleotide encoding a polypeptide encoding the imine reductase and is competent to express imine reductase activity. The available microorganisms include, the microorganisms listed below:
bacterial strains for which host-vector systems are available, such as those belonging to:
genus *Escherichia,*
genus *Bacillus,*
genus *Pseudomonas,*
genus *Serratia,*
genus *Brevibacterium,*
genus *Corynebacterium,*
genus *Streptococcus,* and
genus *Lactobacillus;*
actinomycetes for which host-vector systems are available, such as those belonging to:
genus *Rhodococcus,* and
genus *Streptomyces;*
yeasts for which host-vector systems are available, such as those belonging to:
genus *Saccharomyces,*
genus *Kluyveromyces,*
genus *Schizosaccharomyces,*
genus *Zygosaccharomyces,*
genus *Yarrowia,*
genus *Trichosporon,*
genus *Rhodosporidium,*
genus *Pichia,* and
genus *Candida;* and
molds for which host-vector systems are available, such as those belonging to:
genus *Neurospora,*
genus *Aspergillus,*
genus *Cephalosporium,* and
genus *Trichoderma.*

Preparation of transformants and construction of recombinant vectors compatible to each host can be achieved by using conventional techniques of molecular biology, bioengineering, and genetic engineering (for example, Sambrook *et al.,* Molecular Cloning, Cold Spring Harbor Laboratories). To obtain expression of the genes of the present invention for imine reductases that use NADPH as an electron donor in a microorganism, the DNA should be first inserted into a plasmid or phage vector that is stable in the microorganism and which will permit transcription and translation of the genetic information.

To achieve this, a promoter, which is a regulatory unit for transcription and translation, is inserted at the 5'-side (upstream) of the DNA chain of the present invention, and more preferably a terminator is also inserted at the 3'-side (downstream) of the DNA. Such promoters and terminators should be known to be functional in the host microorganisms. Such vectors, promoters, terminators, and others, which can be used with various microorganisms, are described in detail in "Fundamental Microbiology (Biseibutsugaku Kiso-kouza) 8: Genetic Engineering, KYORITSU SHUPPAN CO., LTD.", and in particular those to be used with yeasts are described in detail in: Adv. Biochem. Eng. 43, 75-102 (1990); Yeast 8, 423-488 (1992); and others.

For example, for bacterial species belonging to the genus *Escherichia,* in particular, *Escherichia coli,* pBR and pUC plasmids are available as plasmid vectors, and available promoters include those derived from lac (β-galactosidase), trp (tryptophan operon), tac, trc (fusion of lac and trp), and λ phage PL and PR. Available terminators include those derived from trpA, phage, and rrnB ribosomal RNA. Suitable vectors include pSE420D (described in JP-A (Kokai) 2000-189170), which has a modified cloning site derived from the multi-cloning site of the commercially available pSE420 (Invitrogen).

For bacterial species belonging to the genus *Bacillus,* available vectors include pUB 110 plasmids and pC 194 plasmids. The plasmids can be integrated into the chromosome. Promoters and terminators are available from genes including apr (alkaline protease), npr (neutral protease), amy (α-amylase).

For *Pseudomonas putida, Pseudomonas cepacia,* and other species from the genus *Pseudomonas,* host-vector systems have been developed previously. Wide host-range vectors (containing genes required for autonomous replication derived from RSF1010 and others), such as pKT240, are available; and these vectors were constructed from the plasmid TOL which is involved in the degradation of toluene and toluene-containing compounds. The lipase gene (JP-A (Kokai) H05-284973) can provide the promoter and terminator.

For bacterial species belonging to the genus *Brevibacterium,* in particular, *Brevibacterium lactofermentum,* available plasmid vectors include pAJ43 (Gene 39, 281 (1985)). This species can utilize promoters and terminators that are suitable for *E. coli,* without modification.

For bacterial species belonging to the genus *Corynebacterium,* in particular, *Corynebacterium glutamicum,* plasmid vectors such as pCS 11 (JP-A (Kokai) S57-183799) and pCB101 (Mol. Gen. Genet. 196, 175 (1984)) are available.

For bacterial species belonging to the genus *Streptococcus,* plasmid vectors such as pHV1301 (FEMS Microbiol. Lett. 26, 239 (1985)) and pGK1 (Appl. Environ. Microbiol. 50, 94 (1985)) are available.

For bacterial species belonging to the genus *Lactobacillus,* the plasmid vector pAM β1 (J. Bacteriol. 137, 614 (1979)) is available; and this plasmid vector was constructed for bacterial species belonging to the genus *Streptococcus,* and promoters which are suitable for *E. coli* can also be used.

For bacterial species belonging to the genus *Rhodococcus,* plasmid vectors isolated from *Rhodococcus rhodochrous* (J. Gen. Microbiol. 138, 1003 (1992)) are available.

For bacterial species belonging to the genus *Streptomyces,* plasmids can be constructed by the method of Hopwood *et al.* which is described in Genetic Manipulation of Streptomyces: A Laboratory Manual Cold Spring Harbor Laboratories (1985). In particular for *Streptomyces lividans,* available plasmid vectors are pIJ486 (Mol. Gen. Genet. 203, 468-478 (1986)), pKC1064 (Gene 103, 97-99 (1991)), and pUWL-KS (Gene 165, 149-150 (1995)). Furthermore, the same plasmids can be used for *Streptomyces virginiae* (Actinomycetol. 11, 46-53 (1997)).

For fungal species belonging to the genus *Saccharomyces,* in particular, *Saccharomyces cerevisiae,* YRp plasmids, YEp plasmids, YCp plasmids, and YIp plasmids are available. Integration vectors (*e.g*., EP 537456) which use homologous recombination with multicopy ribosomal DNA on the chromosome are highly advantageous since a gene of interest can be introduced in multiple copies using the vector, and the integrated gene is stable in the host. Available promoters and terminators are derived from alcohol dehydrogenase (ADH), glyceraldehyde-3-phosphate dehydrogenase (GAPDH), acid phosphatase (PHO), β-galactosidase (GAL), phosphoglycerate kinase (PGK), enolase (ENO), and others.

For fungal species belonging to the genus *Kluyveromyces,* in particular, *Kluyveromyces lactis,* available plasmid vectors include *Saccharomyces cerevisiae-derived* 2µm plasmids and pKD1 plasmids (J. Bacteriol. 145, 382-390 (1981)), plasmids derived from pGKI1 involved in killer activity, KARS plasmids derived from genes associated with the autonomous replication in fungal species belonging to the genus *Kluyveromyces,* and vector plasmids capable of integrating into the chromosome *via* homologous recombination with ribosomal DNA (*e.g.,* EP 537456). Promoters and terminators derived from ADH, PGK, and other genes are also available.

For fungal species belonging to the genus *Schizosaccharomyces,* available plasmid vectors are: ARS derived from *Schizosaccharomyces pombe* (genes involved in the autonomous replication) and *Saccharomyces cerevisiae-derived* plasmid vectors containing selection markers which complement nutritional requirement (Mol. Cell. Biol. 6, 80 (1986)). In addition, the ADH promoter and others derived from *Schizosaccharomyces pombe* are available (EMBO J. 6, 729 (1987)). In particular, the commercially available pAUR224 (Takara Bio Inc.) can be readily used.

For fungal species belonging to the genus *Zygosaccharomyces,* plasmid vectors derived from the *Zygosaccharomyces rouxii*-derived plasmid pSB3 (Nucleic Acids Res. 13, 4267 (1985)) or the like are available, and available promoters include the PHO5 promoter derived from *Saccharomyces cerevisiae* and the GAP-Zr (glyceraldehyde-3-phosphate dehydrogenase) promoter derived from *Zygosaccharomyces rouxii* (Agri. Biol. Chem. 54, 2521 (1990)).

For *Pichia angusta* (previous name: *Hansenula polymorpha*) belonging to the genus *Pichia,* there are previously developed host-vector systems. *Pichia angusta-derived* genes involved in the autonomous replication (HARS1 and HARS2) can be used as vectors. However, these genes are relatively unstable, and therefore, multicopy integration into the chromosome (Yeast 7, 431-443 (1991)) may be used more advantageously. The AOX (alcohol oxidase) promoter which is inducible with methanol or the like, or the FDH (formate dehydrogenase) promoter can be used for this fungal species. In addition, for *Pichia pastoris* and others, host-vector systems using Pichia-derived genes involved in the autonomous replication (PARS 1 and PARS2) or the like have been developed (Mol. Cell. Biol. 5, 3376 (1985)). Thus, it is possible to use high efficiency promoters such as AOX which is inducible with high density culture and methanol (Nucleic Acids Res. 15, 3859 (1987)).

For *Candida maltosa, Candida albicans, Candida tropicalis, Candida utilis,* and other species belonging to the genus *Candida,* host-vector systems have been developed. *Candida maltosa-derived ARS* has been cloned previously (Agri. Biol. Chem. 51, 51, 1587 (1987)), and thus vectors using this ARS have been developed for *Candida maltosa.* In addition, chromosome integration vectors with high efficiency promoters have been developed for *Candida utilis* (JP-A (Kokai) H08-173170).

The fungal species *Aspergillus niger* and *Aspergillus oryzae,* which belong to the genus *Aspergillus,* have been extensively studied. Plasmids, chromosome integration vectors, and extracellular protease and amylase derived promoters are also available for the fungal species (Trends in Biotechnology 7,283-287 (1989)).

For *Trichoderma reesei,* belonging to the genus *Trichoderma,* a host-vector system has been developed, and an extracellular cellulase gene-derived promoter and others are available (Biotechnology 7, 596-603 (1989)).

Furthermore, various host-vector systems have been developed for plant and animal species, in addition to microorganisms. Large-scale systems for the expression of foreign proteins in insects, in particularly, silkworms (Nature 315, 592-594 (1985)), and plants such as colza, maize, and potato have been developed and are suitable for use.

Microorganisms having the ability to produce imine reductases of the present invention include all bacterial strains that belong to the genus *Streptomyces* and have the ability to produce imine reductases, mutants and variants thereof, and transformed strains which gain the ability to produce the enzyme of the present invention prepared by genetic engineering techniques.

Methods for producing optically active amine derivatives of the present invention comprise a step of contacting a substrate compound represented by formula (I) or (III) with enzymatically active materials having an imine reductase activity, which is the ability to stereoselectively reduce the substrate compound under conditions that enable the reduction of the substrate compound by the enzymatically active materials. Herein, the "enzymatically active materials" refers to materials that are derived from a microorganism having the ability to stereoselectively reduce the substrate compound represented by formula (I) or (III) and retain the ability. Specifically, for example, the components described below are included in the enzymatically active materials of the present invention, as long as they retain the imine reductase activity.
(i) Microorganisms having the ability to stereoselectively reduce the substrate compound represented by formula (I) or (III); and
(ii) processed products of cells of the microorganisms described in (i).

The microorganisms of the present invention may be culture or microbial cells. The culture refers to a condition in which the microorganisms exist with the medium that supports the survival and growth of the microorganisms. For example, liquid medium containing microorganisms is the culture. Meanwhile, as used herein, "microbial cell" is a term that refers to a microorganism itself. Microbial cells are cells of a microorganism collected from the culture. For example, microbial cells of a microorganism can be collected from liquid medium by centrifugation. The components of medium may be removed from the surface of microbial cells by washing the microbial cells collected from the culture.

Furthermore, in an embodiment, the enzymatically active materials of the present invention also include imine reductases that can be isolated from the microorganisms described above in (i). The imine reductases immobilized onto carriers are also included in the enzymatically active materials, as long as they retain the imine reductase activity.

Without any modification or after immobilization, the enzymatically active materials of the present invention may be contacted with substrate compounds. There are various known methods for immobilizing microbial cells or processed products thereof. Such known methods for immobilizing microbial cells or processed products thereof include, for example:
polyacrylamide method;
sulfur-containing polysaccharide method (κ-carrageenan gel method, etc.);
alginic acid gel method;
agar gel method; and
ion exchange resin method.

Alternatively, microorganisms, processed products thereof, or the enzymes may be reacted by contacting them with substrate compounds in a membrane bioreactor using ultrafiltration membrane or the like.

The stereoselective imine reduction by the microorganisms of the present invention can be carried out by the following procedure. The microorganisms are cultured under the above-described culture conditions suitable for inducing the enzyme; an imine derivative is added as a reaction substrate to the prepared culture liquid, microbial cells collected from the culture liquid, processed product thereof, or the enzyme; and the mixture is incubated. Alternatively, an imine derivative which serves as a reaction substrate during culture is added at the start of culture of microorganism described above. Thus, the stereoselective imine reduction occurs during culture.

Such reaction conditions include, for example, those shown below:
pH: 4.0 to 9.0, preferably 6.0 to 8.0;
temperature: 15 to 50°C, preferably 20 to 40°C; and
reaction time: contacting for four hours to seven days.

In general, contamination of impurity in the medium of the reaction system can be minimized by performing the culture of the microorganisms and reaction individually. This can be advantageous when the product is purified after reaction.

Furthermore, a more efficient enzymatic reduction reaction can be expected by performing the reaction in the presence of sugars, organic acids, amino acids, or alcohols as a reduction energy source. Compounds that are added as reduction energy sources can be added according to the amount of imine derivative as a reaction substrate. More specifically, the following can be used as reduction energy sources.
sugars: glucose, glucose-6-phosphate, sucrose, etc.
organic acids: malic acid, citric acid, formic acid, etc.
amino acids: alanine, glutamic acid, lysine, etc.
alcohols: ethanol, isopropanol, etc.

In addition, inorganic compounds such as phosphorous acid can be used as reduction energy sources in some cases.

The reduction reaction of the present invention proceeds in the presence of a hydrogen donor. Accordingly, the condition that enables the reduction of a substrate compound in the present invention is achieved by incubating the substrate compound with enzymatically active materials in the presence of a hydrogen donor. Any hydrogen donor can be used as long as it supports the reduction reaction of the present invention. Preferred hydrogen donors of the present invention include NADPH and NADH. NADPH is preferably used in combination with an imine reductase derived from *Streptomyces* sp. GF3587, which was successfully isolated by the present inventors, homologues thereof, or an enzymatically active material thereof.

The regeneration of NADPH from NADP⁺, which is generated from NADPH accompanying the reduction reaction described above, can be achieved using the NADP⁺-reducing ability of a microorganism (glycolytic system, the assimilation pathway of methylotroph C1 compound, etc.). The NADP⁺-reducing ability can be enhanced by adding glucose, ethanol, or the like to the reaction system. Alternatively, the regeneration can be achieved by adding to the reaction system a microorganism that has the ability to generate NADPH from NADP⁺, processed product thereof, or enzyme thereof. For example, NADPH can be regenerated by using microorganisms having glucose dehydrogenase, alcohol dehydrogenase, amino acid dehydrogenase, organic acid dehydrogenase (malate dehydrogenase, etc.), or such, processed products thereof, or purified or partially purified enzymes thereof. These components which constitute the reaction required for the NADPH regeneration can be added to the reaction system for producing optically active amines of the present invention, added after immobilization, or contacted through an NADPH-exchangeable membrane.

Alternatively, the additional reaction system for NADPH regeneration may be unnecessary in some cases when viable microbial cells of a microorganism transformed with a recombinant vector carrying a DNA of the present invention are used in the above-described methods for producing optically active amines. More specifically, efficient reduction can be achieved without adding NADPH regenerating enzyme by using transformed microorganisms having a strong activity of regenerating NADPH. Furthermore, more efficient imine reduction reaction can be achieved by co-expressing an imine reductase with an NADPH regenerating enzyme as follows. A DNA encoding an imine reductase of the present invention is introduced into a host, along with a gene available for NADPH regeneration, such as a gene encoding glucose dehydrogenase, alcohol dehydrogenase, amino acid dehydrogenase, or organic acid dehydrogenase (malate dehydrogenase, etc.). To avoid incompatibility when introducing two or more of such genes into hosts, the respective genes are individually inserted into different recombinant vectors whose replication origins are different from one another, and hosts are transformed with these vectors. Alternatively, two genes are inserted into a single vector, and both or one of the genes are integrated into the chromosome.

When multiple genes are inserted into a single vector, regulatory regions for expression such as the promoter and terminator may be linked to each gene. Alternatively, the genes may be expressed as an operon containing multiple cistrons, such as the lactose operon.

For example, glucose dehydrogenases derived from the species that belongs to *Bacillus subtilis* or *Thermoplasma acidophilum* can be used as an NADPH regenerating enzymes. Specifically, preferred recombinant vectors include pSGSRIR1, which contains the genes encoding the imine reductase and the glucose dehydrogenase derived from *Bacillus subtilis* as inserts.

When there are changes in the pH of a reaction solution in association with the degradation of amine derivatives, the reaction can be continued by regulating pH within a certain range using an appropriate acid or base and by keeping good reactivity.

When viable microbial cells are used in the present invention, in some cases the reaction period can be shortened by adding a detergent to the reaction mixture. Detergents to be used for this purpose are not particularly limited, as long as they increase the permeability of the cell wall of viable microbial cells. Such detergents include cetylpyridinium bromide, cetyltrimethylammonium bromide, Triton X-100, paraisooctylphenyl ether, Tween80, and Span 60. The detergents are preferably used at about 0.0001 to 1% in the reaction mixture.

Alternatively, the same effect can be expected by adding an organic solvent to the reaction solution in some cases. Detergents to be used for this purpose are not particularly limited, as long as they increase the permeability of the cell wall of viable microbial cells. Such organic solvents include toluene and xylene. The organic solvents are preferably used at about 0.0001 to 1 % in the reaction solution.

Alternatively, instead of making additions to the reaction solution, it is possible to use microbial cells pre-treated with water or buffer containing a detergent or organic solvent after cell harvest to increase the permeability of the cell wall.

Imine derivatives which are used as a reaction substrate of the present invention can be used at a concentration adequate for efficient production of the target product. In some cases, imine derivatives are toxic to microbial cells or the enzyme that catalyzes the reaction. Thus, it is noteworthy that reactions with a high concentration of the imine derivatives may not necessarily result in efficient production. The concentration of imine derivatives in the reaction mixture ranges from, for example, 0.05 to 50%w/v, preferably 0.1 to 10%w/v. Imine derivatives can be added by any method such as batch method, fed-batch method, and feed method. Alternatively, instead of adding the substrate imine, a ketone and an amine, which are raw materials for imine, are added to generate an imine in the reaction solution. The generated imine can serve as substrate.

Product quality can be improved by treating imine derivatives to be added under an inactive gas atmosphere to prevent coloration. With such expected effect, raw materials can also be added to the reaction mixture under inactive gas atmosphere. Furthermore, coloration can be prevented by performing the enzyme reaction under an inactive gas atmosphere. Such inactive gas used herein includes, for example, nitrogen, argon, and helium. In particular, nitrogen is an inactive gas that is easy to obtain. Alternatively, argon does not readily diffuse out because its specific gravity is higher than that of air. Thus, argon is operationally advantageous in that the inactive gas atmosphere can be maintained even in an open operation.

The present invention includes the step of collecting optically active amine derivatives obtained by contacting substrate compounds with an enzymatically active material.

Specifically, optically active amine derivatives obtained *via* stereoselective reduction of imine derivatives can be isolated from the reaction solution by the combined use of various chromatographies, crystallization, and the like. Optically active amine derivatives accumulated in the reaction solution can be extracted, for example, by using the following solvents:
ethyl acetate;
butyl acetate;
toluene;
xylene;
hexane;
heptane;
methylisobutylketone;
methyl-*t-*butyl ether; and
organic solvents such as halogen series.

The extracted amine derivatives can be further purified by the chromatographies described below. Before purification, treatments such as washing with acid or alkali, and dehydration with a dehydrating agent can be used in combination if needed.
ion exchange chromatography
adsorption chromatography

For example, after insoluble materials such as microbial cells are removed from the reaction mixture by centrifugation or membrane filtration, extraction is performed using an organic solvent such as ethyl acetate under an alkaline condition. Optically active amine derivatives can be collected by concentrating the extract under reduced pressure.

Alternatively, insoluble materials such as microbial cells are removed from the solution, and the solution is adsorbed onto ion exchange resins or the like which can adsorb amine derivatives. A high concentration solution can be easily obtained by eluting the amine derivatives with alkali such as ammonia water, aqueous solution of sodium hydroxide, methanol solution of sodium hydroxide, or ethanol solution of sodium hydroxide.

Alternatively, after solvent extraction, the extract is converted into hydrochloride salt by treating it with hydrochloric acid to collect the optically active amine derivative as crystal. To improve the yield, the solvent may be concentrated under reduced pressure or replaced with a solvent suitable for crystallization.

Alternatively, in some cases the optical purity of optically active amine derivatives can be increased by preparing them as crystallized salts with acid. Such acids for purifying the salts include mineral acids such as hydrochloric acid and sulfuric acid, and organic acids such as mandelic acid, citric acid, and tartaric acid. The salts of amine derivatives obtained by the methods described above are dissolved in strong acid or strong alkali, and then neutralized with an appropriate alkali or acid to achieve neutralization crystallization. This can readily separate the amine derivatives from other impurities. For example, mineral acids such as hydrochloric acid and sulfuric acid can be used as strongly acidic solution to dissolve the salts of amine derivatives. Meanwhile, aqueous solutions of NaOH and ammonia water can be used as strong alkaline solution.

In addition, amine derivatives can be easily separated from other impurities by heating the aqueous solution, and then cooling to re-crystallize. The amine derivatives can be further purified to high purity by conducting silica gel chromatography using an appropriate developing solvent after dissolving the obtained crystals. Such developing solvents for the silica gel chromatography include, for example, butanol, acetic acid, mixed solvents thereof with water.

All prior art documents cited herein are incorporated herein by reference.

### Examples

Hereinbelow, the present invention will be specifically described with reference to the Examples, but it is not to be construed as being limited thereto. In the tables, "GF" in the bacterial strain names indicates that the strains are held by Gifu University, National University Corporation.

### [Example 1] Screening

Liquid culture medium (pH was adjusted at 7.3) containing 4 g/l yeast extract, 10 g/l malt extract, 4 g/l glucose, 5 mg/l ferric sulfate heptahydrate, and 1 g/l 2-methyl-1-pyrroline was prepared and aliquoted (4 ml) into 18 mm diameter test tubes. The tubes were sterilized by heating at 121 °C for 15 minutes in an autoclave. Various microorganisms were each inoculated to the tubes with a platinum loop. The tubes were incubated at 28°C while shaking for two to six days.

The resulting culture media were centrifuged to collect the microbial cells. 25 mM potassium phosphate buffer (pH 7.0) containing 10 mM 2-methyl-1-pyrroline was added to the cells. The cells were reacted at 25°C while shaking for 24 hours. After the reaction, the cells were removed by centrifugation. Then, TLC analysis was carried out using silica gel (developing solvent: 1-butanol/acetic acid/water = 2/1/1), followed by detection using ninhydrin spray. The reaction mixtures of four microorganism strains shown in Table 1, which successfully generated 2-methyl-1-pyrroline, were derivatized with GITC and the optical purity of 2-methyl-1-pyrroline was determined by liquid chromatography. The analysis result is shown in Table 1. The result demonstrated production of (R)-2-methylpyrrolidine.

**[Table 1]**

| Strain Name | Cumulative Dose (mM) | Optical Purity (% ee) |
|---|---|---|
| | | |
| GF3501 | 2.10 mM | 71.6 (R) |
| GF3585 | 1.00 mM | 79.8 (R) |
| GF3587 | 4.20 mM | 99.1 (R) |
| GF4415 | 1.29 mM | 89.6 (R) |
| | | |

### [Example 2] Identification of GF3587

The microbiological features of GF3587 are summarized in Table 2.

**[Table 2]**

| **SIID5767-02** | | | |
|---|---|---|---|
| 1. MACROSCOPIC OBSERVATION | | | |
| GROWING CONDITION ON EACH MEDIUM | | | CULTURE AT 30°C FOR 3 DAYS OR MORE |
| **ISP2** | | | |
| | COLONY SIZE | | φ**1.0-2.0 mm** |
| | SHAPE OF COLONY SURFACE | | WRINKLE SHAPED |
| | COLONY COLOR | | |
| | | SURFACE (AERIAL HYPHA) | PALE YELLOW |
| | | REAR SURFACE (BASAL HYPHA) | PALE YELLOW |
| | WATER-SOLUBLE PIGMENT PRODUCTITON | | - |
| **ISP3** | | | SURFACE: OCHER REAR SURFACE: OCHER |
| **ISP4** | | | SURFACE: PALE YELLOW REAR SURFACE: PALE YELLOW |
| **ISP5** | | | SURFACE: YELLOW REAR SURFACE: YELLOW |

| 2. MICROSCOPIC OBSERVATION | | | |
|---|---|---|---|
| SHAPE OF AERIAL HYPHA | | | BRANCHED |
| | | | WIDTH0.9-1.1mm |

| 3. PHYSIOLOGICAL AND BIOCHEMICAL PROPERTY | | | |
|---|---|---|---|
| GELATIN LIQUEFACTION | | | - |
| STARCH HYDROLYSIS | | | + |
| NITRATE-REDUCING REACTION | | | - |
| PEPTONZATION/COAGULATION OF SKIM MILK | | | - |
| RANGE OF GROWING TEMPERATURE(°C) | | | |
| | | **20** | + |
| | | **25** | + |
| | | **30** | + |
| | | **37** | + |
| | | **45** | - |
| SALT TOLERANCE(%) | | | |
| | | **4.0** | + |
| | | **7.0** | - |
| | | **10.0** | - |
| | | **13.0** | - |
| CARBON SOURCE AVAILABILITY | | | |
| | | ISP MEDIUM No. 9 | - |
| | | GLUCOSE | + |
| | | L-RHAMNOSE | + |
| | | D-MANNITOL | + |
| | | D-FRUCTOSE | + |
| | | L-ARABINOSE | - |
| | | RAFFINOSE | - |
| | | SUCROSE | - |
| | | D-XYLOSE | + |
| | | INOSITOL | - |
| MELANIN-LIKE PIGMENT PRODUCTION | | | |
| | | ISP MEDIUM No. 6 | - |
| | | ISP MEDIUM No: 7 | - |
| +: POSITIVE -: NEGATIVE | | | |

Then, GF3587 was identified by 16S rDNA analysis. The nucleotide sequence of 16S rDNA was determined using the MicroSeq Full Gene 16S rDNA PCR/Sequencing kit (ABI). The analysis was performed according to the procedure described in the instruction manual of the kit. The determined nucleotide sequence is shown in SEQ ID NO: 1. The nucleotide sequence of SEQ ID NO: 1 was searched against APOLLON DB (product name; TechnoSuruga Laboratory) by BLAST. The result showed that the sequence exhibited 99.9% identity to the 16S rDNA sequences of the following bacterial strains:
*Streptomyces luridiseabiei* S63;
*S. griseorubiginosus* NBRC 13047;
*S. alboviridis* NBRC 13013;
*S. fulvorobeus* NBRC 15898; and
*S. microflavus* NBRC 13062.

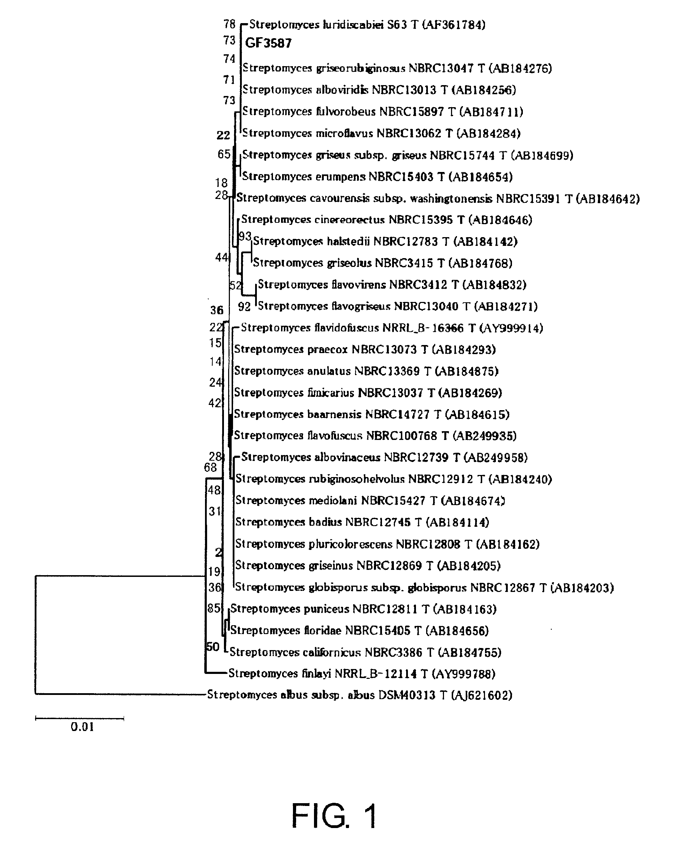

A molecular phylogenetic tree was constructed based on the result and is shown in Fig. 1. The result of cluster analysis suggested that GF3587 belongs to any one of the species described below. Thus, the GF3587 bacterium was identified to belong to *Streptomyces* sp.
*Streptomyces griseorubiginosus;*
*S. alboviridi;*
*S. fulvorobeus;* or
*S. microflavus.*

### [Example 3] Identification of GF3501

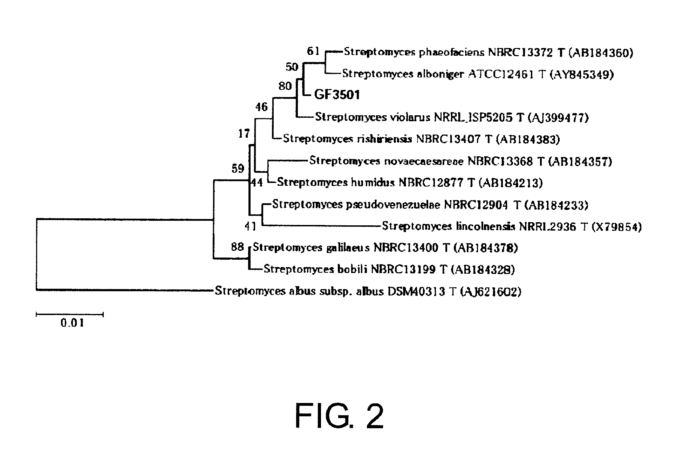

The nucleotide sequence of 16S rDNA of GF3501 was analyzed using the MicroSeq 500 16S rDNA Bacterial Identification PCR kit (ABI). The analysis was performed according to the procedure described in the instruction manual of the kit. The result is shown in SEQ ID NO: 2. The nucleotide sequence of SEQ ID NO: 2 was searched against APOLLON DB by BLAST. The result showed that the standard strain that exhibits the highest identity (99.4%) was *Streptomyces rishiriensis* NBRC 13407. A molecular phylogenetic tree was constructed based on the result of identity search and is shown in Fig. 2. From the result described above, GF3501 was identified to belong to *Streptomyces* sp.

### [Example 4] Identification of GF3585

The nucleotide sequence of 16S rDNA of GF3585 was analyzed using the MicroSeq 500 16S rDNA Bacterial Identification PCR kit (ABI). The analysis was performed according to the procedure described in the instruction manual of the kit. The result is shown in SEQ ID NO: 3. The nucleotide sequence of SEQ ID NO: 3 was searched against APOLLON DB by BLAST. The result showed that standard strains that exhibit the highest identity (99.8%) were the following strains.
*Streptomyces luridiscabiei* S63,
*S. microflavus* NBRC 13062,
*S. praecox* NBRC 13073,
*S. halstedii* NBRC 12783,
*S. griseorubiginosus* NBRC 13047,
*S. griseolus* NBRC 3415,
*S. anulatus* NBRC 13369,
*S. alboviridis* NBRC 13013,
*S. fulvorobeus* NBRC 15897,
*S. griseus* subsp. *griseus* NBRC 15744

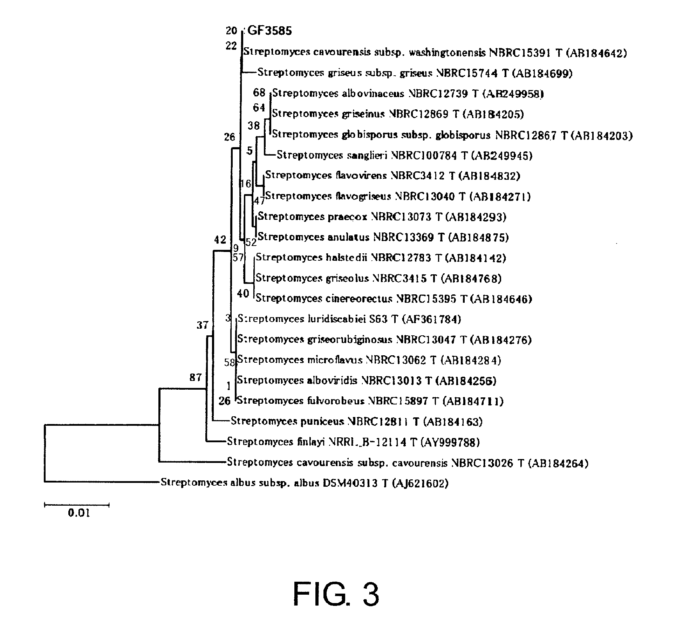

A molecular phylogenetic tree was constructed based on the result of identity search and is shown in Fig. 3. From the result described above, GF3585 was identified as *Streptomyces* sp. close to *Streptomyces cavourensis* subsp. *washintonesis.*

### [Example 5] Identification of GF4415

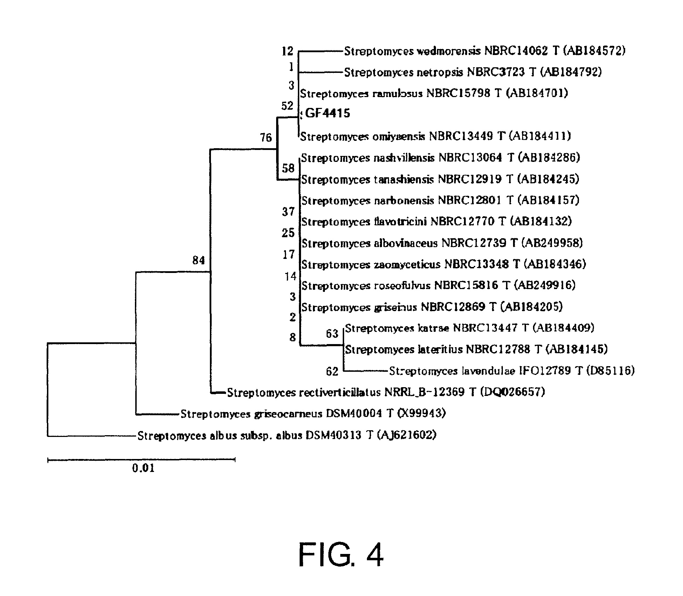

The nucleotide sequence of 16S rDNA of GF4415 was analyzed using MicroSeq 500 16S rDNA Bacterial Identification PCR Kit (ABI). The analysis was performed according to the procedure described in the instruction manual of the kit. The result is shown in SEQ ID NO: 4. The nucleotide sequence of SEQ ID NO: 4 was searched against APOLLON DB by BLAST. The result showed that the standard strain that exhibits the highest identity was *Streptomyces omiyaensis* NBRC 13449. Its identity was 95.6% and is lower than 97%, which is used as an index of different species. A molecular phylogenetic tree was constructed based on the result of identity search and is shown in Fig. 4. From the result described above, GF4415 was identified to belong to *Streptomyces* sp.

### [Example 6] Enzymatic reaction

The cells of *Streptomyces* sp. GF3587 were cultured according to Example 1, and then crushed with glass beads. A crude enzyme solution was prepared by removing intact microbial cells and cell debris by centrifugation. 100 µl of the crude enzyme solution was added to 100 mM phosphate buffer (pH 7.0) containing 10 mM 2-methyl-1-pyrroline and 10 mM NADH or NADPH. During incubation of the mixture at 30°C, absorbance was measured at 340 nm to monitor the decrease of NAD(P)H. The enzymatic activity was determined by this assay. The result showed that when the activity determined using NADPH as a coenzyme is taken as 100%, the activity was 7% with NADH. This finding demonstrates that the imine reductase responsible for the reduction of 2-methyl-1-pyrroline mainly uses NADPH as a coenzyme.

### [Example 7] Microbial cell reaction 1

Liquid culture medium containing 4 g/l NZ amine, 10 g/l malt extract, 8 g/l glucose, and 5 mg/l ferric sulfate heptahydrate (pH was adjusted at 7.3) was aliquoted (40 ml) into shaking flasks. The flasks were sterilized by heating at 121 °C for 15 minutes in an autoclave. *Streptomyces* sp. GF3587 was inoculated to the flasks with a platinum loop. The flasks were incubated at 28°C while shaking for 24 hours.

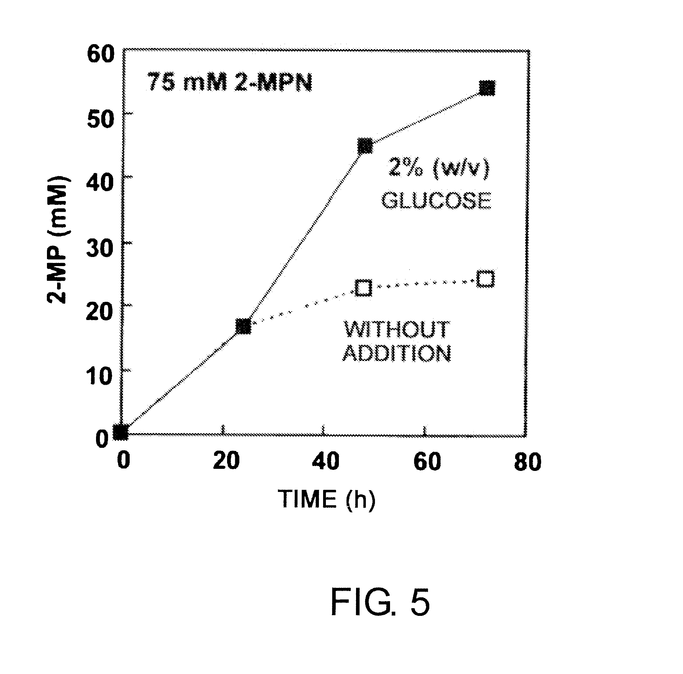

The resulting culture media were centrifuged to collect the microbial cells. 75 mM 2-methyl-1-pyrroline and 100 mM phosphate buffer (pH 7.0) were added to the cells. The cells were reacted at 25°C while shaking. After 24 hours of reaction, 2% glucose was added to one reaction mixture, while the other was incubated continuously without adding glucose. The reaction result is shown in Fig. 5. The efficiency of 2-methyl-1-pyrroline reduction reaction was evidently improved by adding glucose. The concentration of produced (R)-2-methylpyrrolidine reached 54 mM after 72 hours of incubation.

### [Example 8] Microbial cell reaction 2

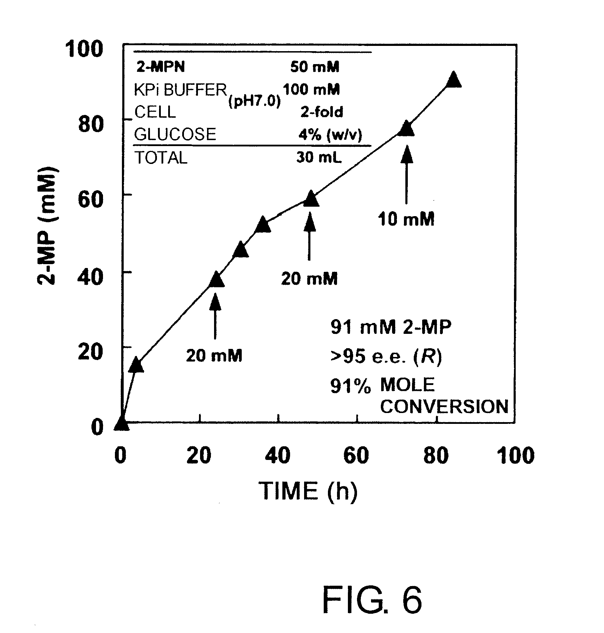

*Streptomyces* sp. GF3587 was cultured and the microbial cells were harvested according to Example 7. The collected microbial cells were suspended in 100 mM potassium phosphate buffer (pH 7.0) containing 50 mM 2-methyl-1-pyrroline and 4% glucose. The suspension was incubated at 25°C while shaking. After 24, 48, and 72 hours of incubation, 2-methyl-1-pyrroline was added at 20 mM, 20 mM, and 10 mM, respectively, and the accumulation of (R)-2-methylpyrrolidine as reaction product was observed. The reaction result is shown in Fig. 6. The concentration of accumulated (R)-2-methylpyrrolidine reached 91 mM after 84 hours of incubation. The optical purity of produced (R)-2-methylpyrrolidine was 95%ee or higher. The optical purity was determined by the following method.

The ingredients described below were added to 50 µl of reaction solution, and the resulting mixture was incubated at 40°C for one hour. The produced amine was derivatized with GITC, and then isolated by HPLC and quantified. 50 µl of 2 mg/ml triethylamine; and 100 µl of 8 mg/ml 2,3,4,6-tetra-O-acetyl-β-D-glucopyranosyl isothiocyanate (GITC).

The conditions of HPLC were as follows:
- HPLC column: Wakosil II 5C18 (4.6 mm x 150 mm) from Wako Pure Chemical Industries
- elution buffer: 10 mM phosphate buffer (pH 2.5)/methanol = 55/45
- flow rate: 1 ml/min
- detection: UV absorbance at 254 nm

Under the conditions described above, (R)-2-methylpyrrolidine was eluted at 26.3 minutes, while (S)-2-methylpyrrolidine was eluted at 24.9 minutes. The optical purity of each eluted fraction was determined based on the UV absorbance at 254 nm.

### [Example 9] Purification of imine reductase from GF3587

GF3587 was cultured and the microbial cells were harvested according to the method described in Example 7. The prepared microbial cells were suspended in a standard buffer containing 100 mM potassium phosphate buffer (pH 7.0) and 1 mM dithiothreitol (DTT), and crushed by sonication.

The lysate of microbial cells was fractionated with 40 to 70% ammonium sulfate. The prepared enzyme was dialyzed against a buffer containing 10 mM potassium phosphate buffer (pH 7.0) and 1 mM DTT, and then loaded onto a DEAE-Sephacel column (2.0 x 14 cm) equilibrated with the same buffer. The column was eluted stepwise with potassium chloride. Imine reductase was eluted with 100 mM potassium phosphate buffer containing 100 mM potassium chloride.

The active fractions were collected and concentrated. Then, the concentrated sample was loaded onto a Phenylsepharose column (1.0 x 14 cm) saturated with 30% sulfuric acid.

After the column was washed with the same buffer, the enzyme was eluted by gradually decreasing the ammonium sulfate concentration. The imine reductase was found in the fraction eluted with the buffer that does not contain ammonium sulfate. The purification was summarized in Table 3.

**[Table 3]**

| Purification of imine reductase from GF3587 | | | |
|---|---|---|---|
| Purification Step | Total Protein Amount (mg) | Total Activity (U) | Relative Activity (U/mg) |
| | | | |
| Cell-free Extract | 717 | 112 | 0.157 |
| 40-70% Ammonium Sulfate Fraction | 268 | 87.6 | 0.327 |
| DEAE-Sephacel | 22.5 | 45.8 | 2.04 |
| Phenylsepharose | 2.2 | 6.6 | 3.00 |
| | | | |

Next, the imine reductase purified from GF3587 was assessed for its physicochemical properties.

### (1) Optimal temperature:

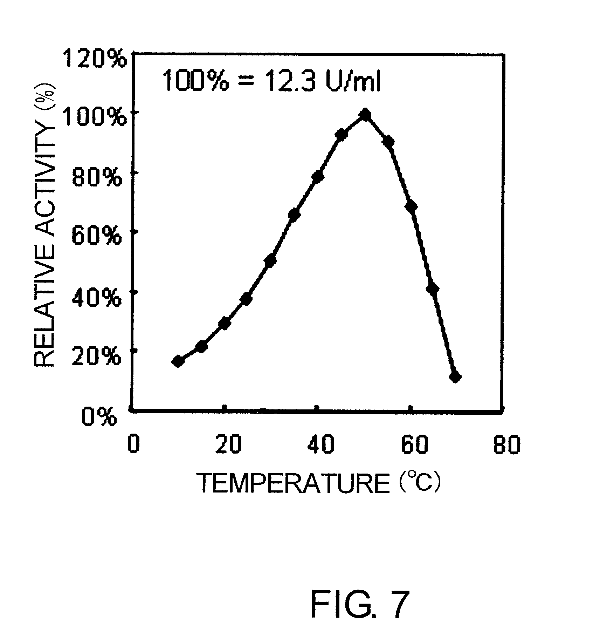

The imine reductase purified from GF3587 was tested for its optimal temperature. 1 ml of reaction solution having the composition described below were incubated at 10 to 70°C to measure the decrease of absorbance (340 nm) of NADPH due to the generation of (R)-2-methylpyrrolidine. After the reaction solutions were pre-incubated at each temperature for five minutes, an imine reductase solution (12.3 U/ml) was added thereto to initiate the reaction. After two minutes, the absorbance of the reaction solutions was measured at 340 nm. The amount of imine reductase that catalyzes the decrease (oxidation) of 1 µmol of NADPH per minute when incubated at 30°C in the reaction solution having the composition described below is defined as 1 U. The result is shown in Fig. 7. This result demonstrated that the optimal temperature of imine reductase purified from GF3587 was 50°C. Furthermore, 80% or more of the maximal activity was found to be retained at 40 to 55°C.

### Composition of reaction mixture:

| | |
|---|---|
| 10 mM 2-methyl-1-pyrroline | |
| 100 mM potassium phosphate buffer (pH 7.0) | |
| 0.1 mM NADPH | |
| 1 µl of imine reductase solution | |
| Total | 1 ml |

### (2) Thermal stability:

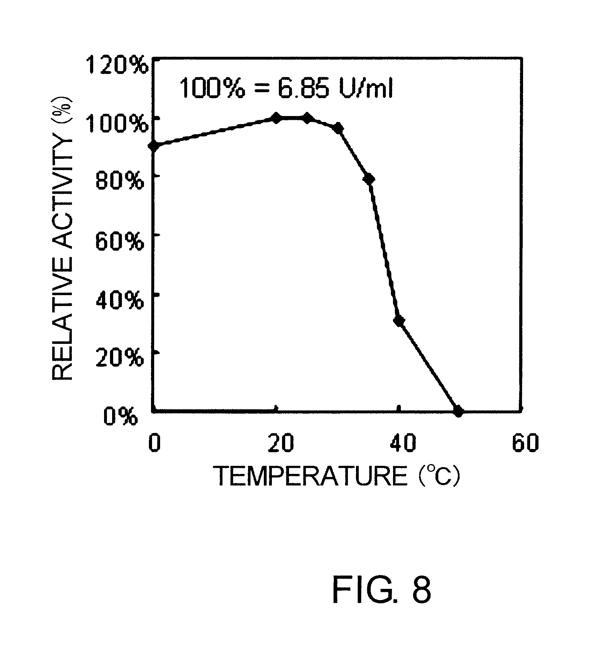

Then, the imine reductase purified from GF3587 was assessed for its thermal stability. First, 6.85 U/ml imine reductase solutions were pre-incubated at 0 to 50°C for 30 minutes, and then cooled on ice for ten minutes. Then, the imine reductase treated at each temperature was added to a reaction solution having the same composition indicated in (1) to initiate the reaction. After two minutes of incubation at 30°C, the reaction mixtures were assayed to measure the decrease in the absorbance (340 nm) of NADPH due to the generation of (R)-2-methylpyrrolidine. The result is shown in Fig. 8. The result showed that the imine reductase purified from GF3587 retained about 80% of the activity up to 35°C but rapidly lost its activity at 40°C or higher temperature.

### (3) Optimal pH:

Furthermore, the imine reductase purified from GF3587 was tested for its optimal pH. The reaction was started by adding an imine reductase (6.82 U/ml) solution to reaction mixtures having the same composition as shown in (1), except that various types of buffer agents were added. The mixtures were incubated at 30°C for two minutes. The buffers used in this experiment and their pH are listed below.

| | |
|---|---|
| 100 mM citrate buffer | : pH 3 to 6 |
| 100 mM sodium acetate buffer | : pH 4 to 5.5 |
| 100 mM potassium phosphate buffer | : pH 6 to 8 |
| 100 mM Tris-HCl buffer | : pH 7.5 to 9 |
| 100 mM glycine-sodium hydroxide buffer | : pH 9 to 11 |

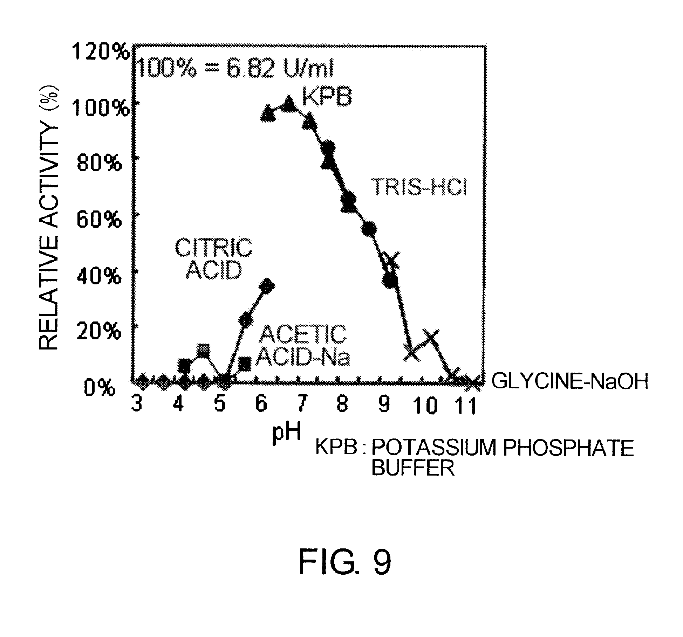

The reaction solutions were assayed to determine the decreases in absorbance (340 nm) of NADPH due to the generation of (R)-2-methylpyrrolidine. The result is shown in Fig. 9. The result showed that the optimal pH of imine reductase purified from GF3587 was 6.5. Furthermore, 80% or more of the maximal activity was retained at pH 6.0 to 7.5.

### (4) pH stability:

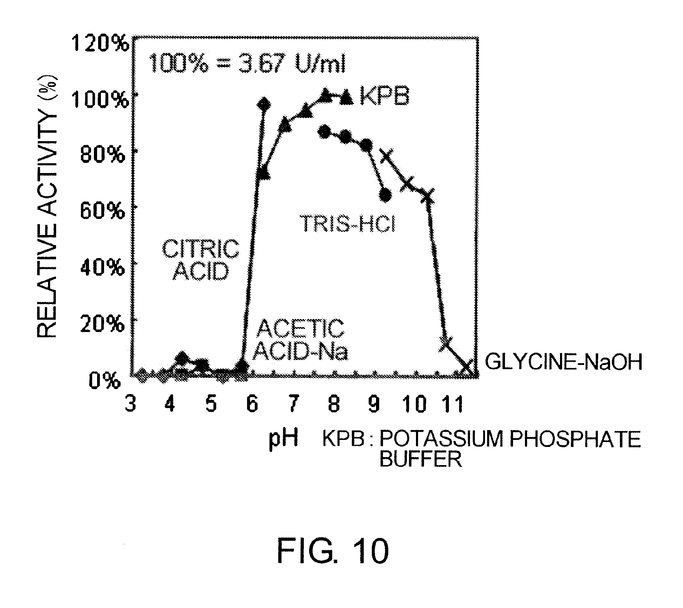

Next, the effect of pH on the stability of the imine reductase purified from GF3587 was assessed. The imine reductase (3.67 U/ml) was dissolved in the same buffer described in (3). The solutions were pre-incubated at 30°C for 30 minutes. The imine reductases treated at each pH were added to a reaction solution having the same composition described in (1) to initiate the reaction. The mixtures were incubated at 30°C for two minutes. The reaction mixtures were assayed to determine the decreases in absorbance (340 nm) of NADPH due to the generation of (R)-2-methylpyrrolidine. The result is shown in Fig. 10. The result showed that the imine reductase purified from GF3587 retained 80% or more of the activity after a 30-minute treatment at 30°C and pH 6.5 to 8.5.

### (5) Substrate specificity:

To assess the substrate specificity of the imine reductase purified from GF3587, the activities of the enzyme towards the compounds listed in Table 4 were compared.

### (6) Determination of molecular weight:

The molecular weight of an imine reductase subunit purified from GF3587 was determined to be about 32,000 by SDS-PAGE. Alternatively, the molecular weight was 65,000 to 70,000 when determined using a gel filtration column of Sephacryl^{™} S-200.

One ml of reaction mixtures having the composition described below were incubated at 30 to 65°C. The reaction mixtures were assayed to determine the decrease in absorbance (340 nm) of NADPH due to the generation of (R)-2-methylpyrrolidine. An imine reductase solution (2.04 U/ml) was added to reaction solutions containing each substrate compound shown in Table 4 to initiate the reaction. The absorbance of each reaction solution was determined at 340 nm after two minutes of incubation.

### Composition of reaction mixture:

| | |
|---|---|
| 0.05 to 25 mM substrate compound | |
| 100 mM potassium phosphate buffer (pH 7.0) | |
| 0.1 mM NADPH | |
| 1 µl of imine reductase solution | |
| Total | 1 ml |

The result showed that of the compounds shown in Table 4, the compounds listed below were reduced by the activity of the imine reductase purified from GF3587. The relative activity shown in the table is defined as relative activity when the enzymatic activity on 2-methyl-1-proline (2-MPN) is taken as 100.

| | |
|---|---|
| 2-methyl-1-pyrroline: | 100% |
| 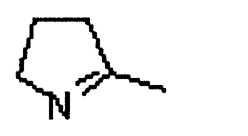 | |
| N-Benzylidenebenzylamine: | 30% |
| 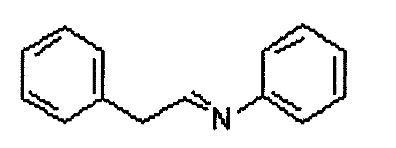 | |
| N-(E)-N-(1-Phenylethylidene)aniline: | 29% |
| 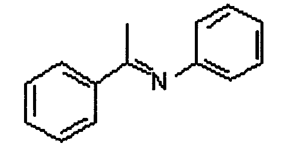 | |
| Dihydrofolic acid: | 7.6% |
| 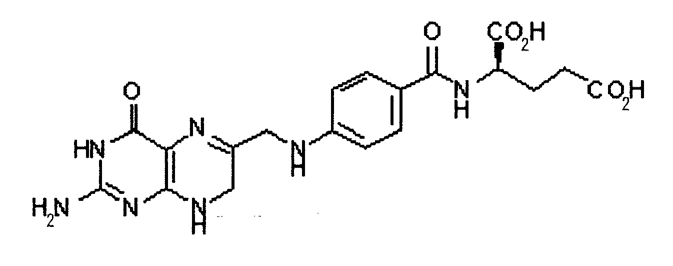 | |

**[Table 4]**

| Substrate Compound (concentration) | Relative Activity |
|---|---|
| 2-Methyl-1-pyrroline (2-MPN) (10 mM) | 100 (2.04 U/mL) |
| N-((E/Z)-N-(1-phenylethylidene) aniline (25 mM) | 29 |
| N-Benzylidenebenzylamine (10 mM) | 30 |
| Acetophenone oxime (1 mM) | 0 |
| Dihydrofolic acid (0.05 mM) | 7.6 |
| 2-Methylpyrazine (10 mM) | 0 |
| 2-Methylpyrimidine (10 mM) | 0 |
| 2-Methylimidazole (10 mM) | 0 |
| 2-Methylthiazole (10 mM) | 0 |
| 2-Methylfuran (10 mM) | 0 |
| 2-Methylthiophene (5 mM) | 0 |
| 1,4,5,6-Tetrahydropyrimidine (10 mM) | 0 |

### [Example 10] Enzymatic activities of GF3587 Streptomyces-related strains

The type strains of the genus *Streptomyces* having 99.9% sequence identity to the 16S rDNA sequence of GF3587 were cultured at 28°C while shaking for 48 to 72 hours in culture medium having the composition described below. The microbial cells were harvested as resting cells.

### Composition of culture medium:

| |
|---|
| 4 g/l NZ amine |
| 20 g/l malt extract |
| 8 g/l glucose |
| 5 mg/l ferric sulfate(II)heptahydrate |
| Volume was filled up to 1 L with tap water (pH 7.3) |

The type strains of the genus *Streptomyces* cultured are the three strains listed below. The nucleotide sequences of 16S rDNA of the strains all have 99.8% sequence identity to that of *Streptomyces* sp. GF3587 (Example 4).
*Streptomyces griseorubiginosus* NBRC 13047
*S. microflavus* NBRC 13062
*S. alboviridis* NBRC 13013

Microbial cells harvested from 8 ml of culture were reacted at 25°C for 48 hours in 4 ml of a reaction solution containing 100 mM potassium phosphate buffer (pH 7.0), 50 mM 2-methyl-1-pyrroline, and 2% glucose. The generated (R)-2-methylpyrrolidine was quantified. As shown in Table 5, the result demonstrated that of the four type strains that have 99.9% sequence identity to the 16S rDNA sequence of GF3587, three strains generated (R)-2-methylpyrrolidine (2-MP) with 98%ee or higher optical purity.

**[Table 5]**

| Strain | Reaction time | 2-MP | Optical Purity |
|---|---|---|---|
| *S. griseorubiginosus* NBRC 13047 | 24 h | 6.4 mM | >98% ee(R) |
| | 48 h | 17.2 mM | >98% ee(R) |
| *S. microflavus* NBRC 13062 | 24 h | 9.0 mM | >98% ee(R) |
| | 48 h | 35.7 mM | >98% ee(R) |
| *S. alboviridis* NBRC 13013 | 24 h | 0.17 mM | >98% ee(R) |
| | 48 h | 0.54 mM | >98% ee(R) |

The three strains of microorganisms used in this experiment are all available from NITE Biological Resource Center (NBRC), National Institute of Technology and Evaluation (http://www.nbrc.nite.go.jp). The contact information of the institute is as follows:
Postal code: 292-0818 2-5-8 Kazusakamatari, Kisarazu-shi, Chiba, Japan Incorporated Administrative Agency National Institute of Technology and Evaluation Department of Biotechnology,
NITE Biological Resource Center (NBRC)

### [Example 11] Partial amino acid sequence of imine reductase

The N-terminal amino acid sequence of the enzyme prepared as described in Example 9 was analyzed using a protein sequencer (Procise 494 HT Protein Sequencer System). The amino acid sequence is shown in SEQ ID NO: 7. Furthermore, a gel fragment containing the imine reductase was excised from the SDS-PAGE gel. After two washes, lysyl endopeptidase was used to perform in-gel digestion at 35°C overnight. The digested peptide was fractionated by reverse phase HPLC (Tosho; TSK gel ODS-80-Ts, 2.0 mm x 250 mm). The peptide was eluted using 0.1 % trifluoroacetic acid with an acetonitrile gradient and collected.

One of the collected peptide peaks was named lep_38. An amino acid sequence of the peptide was analyzed with a protein sequencer (Procise 494 HT Protein Sequencer System). The amino acid sequence of lep_38 is shown in SEQ ID NO: 8.

### [Example 12] Purification of chromosomal DNA from Streptomyces sp. GF3587

GF3587 was cultured and the microbial cells were harvested according to the method described in Example 7. The chromosomal DNA was purified from the microbial cells according to the method described in the reference of J. Dairy Sci., 75, 1186-1191 (1992).

### [Example 13] Cloning of the imine reductase gene core region

Sense and antisense primers were synthesized based on the N-terminal amino acid sequence and the amino acid sequence of lep_38. The nucleotide sequences are shown in SEQ ID NOs: 9 (SsRIR-Nmix) and 10 (SsRIR-IAmix).

Using 50 µl of reaction mixture containing 50 pmol each of the primers, 10 nmol dNTP, 50 ng of chromosomal DNA from *Streptomyces* sp. GF3587, Ex-Taq buffer (Takara Bio), 5% DMSO, and 1 U of Ex-Taq (Takara Bio), PCR was carried out with 30 cycles of denaturation (94°C for 30 seconds), annealing (45°C for 30 seconds), and extension (70°C for one minute and five seconds) in GeneAmp PCR System 9700 (Applied Biosystems).

A part of the PCR reaction mixture was analyzed by agarose gel electrophoresis. A seemingly specific band was detected at around 800 bp. Then, a nucleotide sequence of the DNA fragment was sequenced. The determined nucleotide sequence of the core region is shown in SEQ ID NO: 11.

### [Example 14] Analysis of the nucleotide sequences around the core region of the imine reductase gene

The chromosomal DNA derived from *Streptomyces* sp. GF3587 was digested with restriction enzymes *Bam*HI*, Apa*I, and *Sac*I. Each fragment was cyclized via overnight self-ligation at 16°C using T4 ligase. Then, PCR was carried out using 50 µl of reaction mixture containing 100 pmol each of primers SsRIR-UP (SEQ ID NO: 12) and dSsRIR-DW (SEQ ID NO: 13), 25 ng of cyclized DNA, Ex-Taq buffer (Takara Bio), 5% DMSO, and 1 U of Ex-Taq (Takara Bio) with 30 cycles of denaturation (95°C for 30 seconds), annealing (55°C for 30 seconds), and extension (72°C for seven minutes) in GeneAmp PCR System 9700 (Applied Biosystems). A part of each PCR mixture was analyzed by agarose gel electrophoresis. In association with the digestion of the template DNA with *Bam*HI, *Apa*I, and *Sac*I, DNA fragments of about 6,000 bp, 4,000 bp, and 1,400 bp were detected, respectively. The nucleotide sequences of DNA fragments were analyzed to determine the ORF sequence of the imine reductase gene. The determined DNA sequence is shown in SEQ ID NO: 5, and the deduced amino acid sequence is shown in SEQ ID NO: 6.

### [Example 15] Cloning of the imine reductase gene

Based on the structural gene for the imine reductase, primers SsRIR-ATG1 (SEQ ID NO: 14) and SsRIR-TAA1 (SEQ ID NO: 15) were synthesized to clone only ORF. PCR was carried out using 50 µl of reaction mixture containing 50 pmol each of the primers, 10 nmol dNTP, 50 ng of chromosomal DNA derived from *Streptomyces* sp. GF3587, *Pfu* Turbo-DNA Polymerase buffer (STRATAGENE), 5% DMSO, and 1.9 U of *Pfu* Turbo-DNA Polymerase (STRATAGENE) with 30 cycles of denaturation (98°C for 30 seconds), annealing (60°C for one minute), and extension (72°C for one minute and 10 seconds) in GeneAmp PCR System 9700 (Applied Biosystems).

The resulting DNA fragment was purified and collected using GFX PCR DNA and the Gel Band Purification kit (GE Healthcare). The DNA fragment was double-digested with restriction enzymes *Nco*I and *Xba*I. After agarose gel electrophoresis, a portion containing a band of interest was excised and the DNA fragment was purified using GFX PCR DNA and the Gel Band Purification kit (GE Healthcare).

Using the Takara Ligation kit, the prepared DNA fragment was ligated to pSE420D (a plasmid constructed by modifying the multicloning site of plasmid vector pSE420 from Invitrogen; Japanese Patent Application Kokai Publication No. (JP-A) 2000-189170 (unexamined, published Japanese patent application)) double-digested with *Nco*I and *Xba*I. The *E. coli* JM109 strain was transformed with the resulting vector.

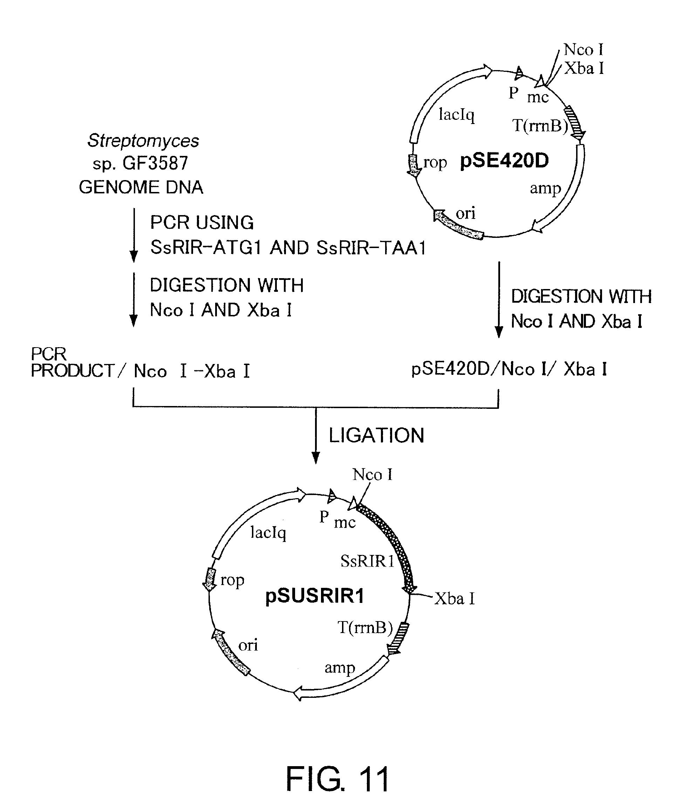

The transformant was grown on an ampicillin-containing LB medium. The plasmid was extracted from the grown transformant. The plasmid was named pSUSRIR1 and confirmed to have the imine reductase gene as an insert. The process of plasmid construction is shown in Fig. 11.

### [Example 16] Assessment of the activity of recombinant imine reductase

The *E. coli* JM109 strain that had been transformed with an imine reductase expression plasmid pSUSRIR1 was cultured in an ampicillin-containing liquid LB medium at 30°C overnight. After adding 0.1 mM IPTG, the cells were further cultured for four hours.

The microbial cells were harvested by centrifugation, and then suspended in 100 mM potassium phosphate buffer (pH 7.0) containing 1 mM DTT. The microbial cells were crushed in Sealed-Type Ultrasonic Cell Disruptor UCD-200TM (Cosmo Bio). After centrifugation, the supernatant was collected as microbial cell extract. Meanwhile, *E. coli* JM109 that does not contain the gene was cultured in LB medium overnight. After adding 0.1 mM IPTG, the cells were further cultured for four hours. Using the same method, the microbial cells were also crushed to prepare microbial cell extract.

Each microbial cell extract was assayed for its imine-reducing activity towards 2-methyl-1-pyrroline. The result is shown in Table 6. The method for assaying the activity is the same as described in Example 6.

**[Table 6]**

| ASSAY RESULT OF RECOMBINANT IMINE REDUCTASE ACTIVITY | | |
|---|---|---|
| Plasmid | IMINE-REDUCING ACTIVITY mU/mg | GLUCOSE DEHYDROGENATION ACTIVITY U/mg |
| NONE | 0 | 0 |
| pSUSRIR1 | 86 | 0 |
| pSGSRIR1 | 158 | 15.1 |
| pSG-SKI1 | 40 | 5.2 |

### [Example 17] Construction of plasmid pSGSRIR1 for coexpressing the imine reductase and Bacillus-derived glucose dehydrogenase

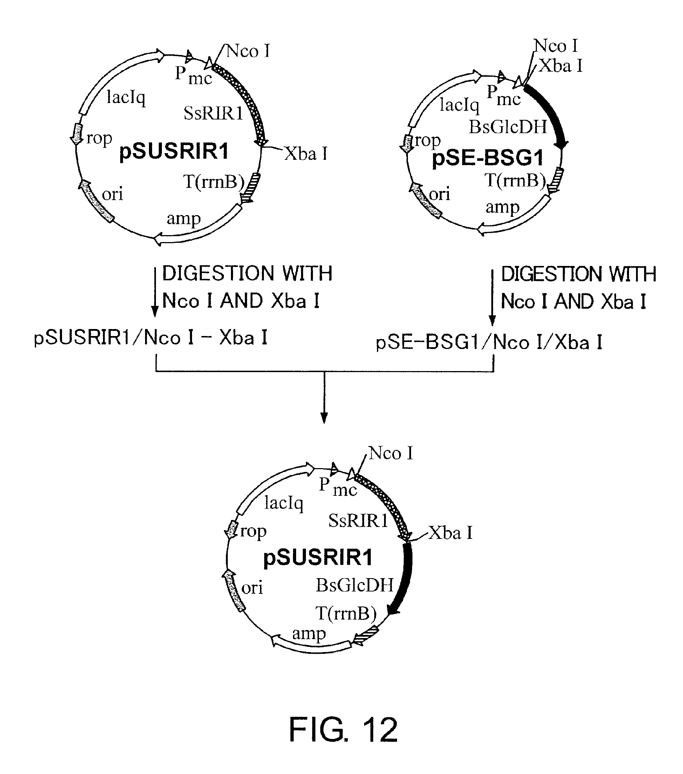

Plasmid pSE-BSG1 (JP-A (Kokai) 2000-189170), which express a *Bacillus*-derived glucose dehydrogenase gene, was double-digested with restriction enzymes *Nco*I and *Xba*I. A DNA fragment containing the imine reductase gene was excised from pSUSRIR1 with the same enzymes. The fragment was ligated to the digested pSE-BSG1 using the Takara Ligation kit to construct the plasmid pSGSRIR1 which can be used to coexpress imine reductase and glucose dehydrogenase. The process of plasmid construction is shown in Fig. 12.

### [Example 18] Coexpression of imine reductase and glucose dehydrogenase in E. coli

The *E. coli* JM109 strain that had been transformed with pSGSRIR1 was cultured in an ampicillin-containing liquid LB medium at 30°C overnight. After adding 0.1 mM IPTG, the cells were further cultured for four hours.

The microbial cells were harvested by centrifugation, and then suspended in 100 mM potassium phosphate buffer (pH 7.0) containing 1 mM DTT. The microbial cells were crushed in Sealed-Type Ultrasonic Cell Disruptor UCD-200TM (Cosmo Bio). The microbial cell lysate was centrifuged, and the supernatant was collected as microbial cell extract. The extract was assayed for its imine-reducing activity towards 2-methyl-1-pyrroline and its glucose dehydrogenase activity. The result is shown in Table 6. The glucose dehydrogenase activity was assayed at 30°C using a reaction solution containing 100 mM potassium phosphate buffer (pH 6.5), 2.5 mM NADP⁺, 100 mM glucose, and microbial cell extract. The amount of enzyme that catalyzes the generation of 1 µmol of NADPH per minute under the-above described conditions is defined as 1 U.

### [Example 19] Purification of chromosomal DNA from Streptomyces kanamyceticus

*Streptomyces kanamyceticus* NBRC 13414 was cultured in *Streptomyces* YEME medium. From the prepared microbial cells, chromosomal DNA was purified according to the method described in the reference of J. Dairy Sci., 75, 1186-1191 (1992).

### [Example 20] Cloning of the imine reductase homologue Q1EQE0

The DNA sequence and amino acid sequence of the deduced protein Q1EQE0 deposited in DDBJ are shown in SEQ ID NOs: 16 and 17, respectively. Primers for PCR (SEQ ID NOs: 18 and 19) were synthesized based on SEQ ID NO: 16. PCR was carried out using 50 µl of reaction mixture containing 50 pmol each of the primers, 10 nmol dNTP, 50 ng of chromosomal DNA derived from *Streptomyces kanamyceticus* NBRC 13414, *Pfu* Turbo-DNA Polymerase buffer (STRATAGENE), 5% DMSO, and 1.9 U of *Pfu* Turbo-DNA Polymerase (STRATAGENE) with 30 cycles of denaturation (98°C for 30 seconds), annealing (60°C for one minute), and extension (72°C for one minute and 10 seconds) in GeneAmp PCR System 9700 (Applied Biosystems). The resulting DNA fragment was purified and collected using GFX PCR DNA and the Gel Band Purification kit (GE Healthcare).

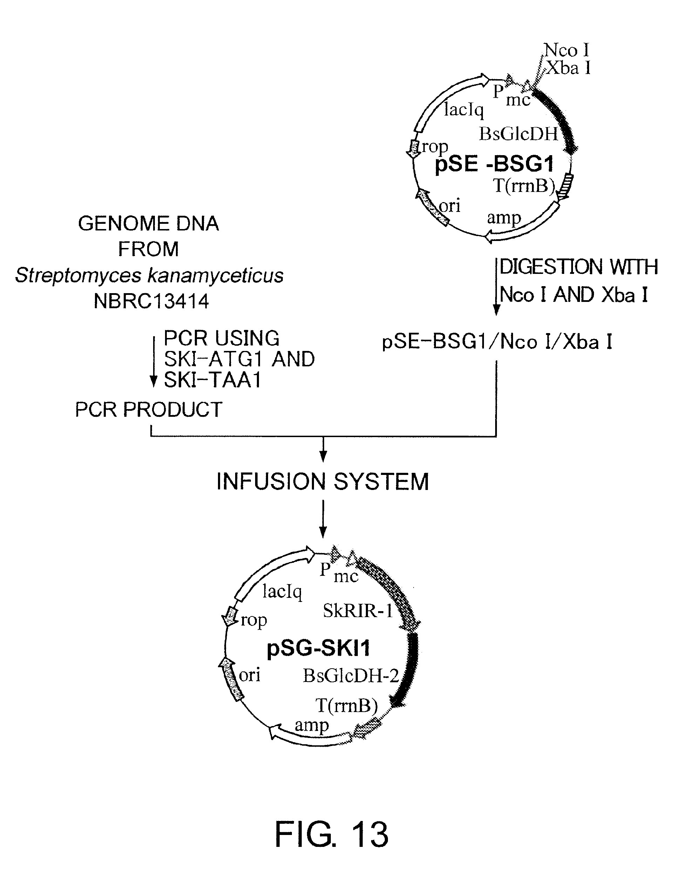

The collected DNA fragment was ligated to the digested pSE-BSG1 (JP-A (Kokai) 2000-189170) double-digested with *Nco*I and *Xba*I using the In-fusion System to construct the plasmid pSG-SKI1 which can be used to coexpress Q1EQE0 and glucose dehydrogenase. The process of plasmid construction is shown in Fig. 13.

### [Example 21] Coexpression of Q1EQE0 and glucose dehydrogenase in E. coli

The *E. coli* JM109 strain that had been transformed with pSG-SKI1 was cultured in an ampicillin-containing liquid LB medium at 30°C overnight. After adding 0.1 mM IPTG, the cells were further cultured for four hours.

The microbial cells were harvested by centrifugation, and then suspended in 100 mM potassium phosphate buffer (pH 7.0) containing 1 mM DTT. The microbial cells were crushed in Sealed-Type Ultrasonic Cell Disruptor UCD-200TM (Cosmo Bio). The microbial cell lysate was centrifuged, and the supernatant was collected as microbial cell extract. The extract was assayed for its imine-reducing activity towards 2-methyl-1-pyrroline and its glucose dehydrogenase activity. The result is shown in Table 6.

### [Example 22] Production of (R)-2-methylpyrrolidine by recombinant E. coli

The *E. coli* JM109 strains that had been transformed with pSGSRIR1 or pSG-SKI1 were cultured in 10 ml of ampicillin-containing liquid LB medium at 30°C overnight. After adding 0.1 mM IPTG, the cells were further cultured for four hours.

The microbial cells were harvested by centrifugation, and then suspended in 10 ml of 100 mM potassium phosphate buffer (pH 7.0) containing 47.5 mM 2-methyl-1-pyrroline and 100 mM glucose. The suspension was reacted at 25°C while shaking overnight. After the reaction, the reaction mixture was assayed to determine the optical purity of the generated 2-methylpyrrolidine, and the concentrations of 2-methyl-1-pyrroline and 2-methylpyrrolidine in the reaction mixture by the same method as described in Example 8. The optical purity of the generated (R)-2-methylpyrrolidine was 99%ee or higher, and the reaction yield was 95% or more. The result is shown in Table 7.

**[Table 7]**

| REACTION RESULT BY RECOMBINANT *E.Coli* | | | | | |
|---|---|---|---|---|---|
| HOST | Plasmid | BEFORE REACTION | AFTER REACTION | | |
| | | 2MPN (mM) | 2MPN | 2MP | |
| | | | (mM) | (mM) | %ee(R) |
| JM109 | pSGSRIR1 | 47.5 | 0.0 | 45.8 | 99.4 |
| JM109 | pSG-SKI1 | 47.5 | 0.8 | 45.1 | 99.6 |

| | | | | | |
|---|---|---|---|---|---|
| 1) 2MNP : 2-METHYL-1-PYRROLINE 2) 2MP : 2-METHYLPYRROLIDINE | | | | | |

### Industrial Applicability

The present invention provides methods for producing optically active R-isomer amine derivatives by stereoselectively reducing imines. Optically active R-isomer amine derivatives produced by the present invention are useful as materials for pharmaceutical agents or such. For example, (R)-2-methylpyrrolidine is a useful compound as a material for pharmaceutical agents.

PCT

0-1 | Form PCT/RO/134 (SAFE)
Indications Relating to Deposited
Microorganism(s) or Other Biological
Material (PCT Rule 13bis) |
0-1-1 | Prepared Using | JPO-PAS
0362
0-2 | International Application No. |
0-3 | Applicant's or agent's file reference | D1-A0803P

1 | The indications made below relate to
the deposited microorganism (s) or
other biological material referred to in
the description on: |
1-1 | Paragraph | 0033
1-3 | Identification of deposit |
1-3-1 | Name or depositary institution | NPMD National Institute of Technology and Evaluation,
Patent Microorganisms Depositary
1-3-2 | Address of depositary institution | 2-5-8 Kazusakamatari Kisarazu-city Chiba 292-0818
Japan
1-3-3 | Date of deposit | 24 June 2008 (24.06.2008)
1-3-4 | Accession Number | NPMD NITE BP-594
1-5 | Designated States for Which
Indications are Made | All designations
2 | The indications made below relate to
the deposited microorganism (s) or
other biological material referred to in
the description on: |
2-1 | Paragraph | 0033
2-3 | Identification of deposit |
2-3-1 | Name or depositary institution | NPMD National Institute of Technology and Evaluation,
Patent Microorganisms Depositary
2-3-2 | Address of depositary institution | 2-5-8 Kazusakamatari Kisarazu-city Chiba 292-0818
Japan
2-3-3 | Date of deposit | 24 June 2008 (24.06.2008)
2-3-4 | Accession Number | NPMD NITE BP-595
2-5 | Designated States for Which
Indications are Made | All designations
3 | The indications made below relate to
the deposited microorganism (s) or
other biological material referred to in
the description on: |
3-1 | Paragraph | 0033
3-3 | Identification of deposit |
3-3-1 | Name or depositary institution | NPMD National Institute of Technology and Evaluation,
Patent Microorganisms Depositary

PCT
3-3-2 | Address of depositary institution | 2-5-8 Kazusakamatari Kisarazu-city Chiba 292-0818
| | Japan
3-3-3 | Date of deposit | 24 June 2008 (24.06.2008)
3-3-4 | Accession Number | NPMD NITE BP-596
3-5 | Designated States for Which
| Indications are Made | All designations
4 | The indications made below relate to
| the deposited microorganism (s) or
| other biological material referred to in
| the description on:
4-1 | Paragraph | 0033
4-3 | Identification of deposit
4-3-1 | Name or depositary institution | NPMD National Institute of Technology and Evaluation,
| | Patent Microorganisms Depositary
4-3-2 | Address of depositary institution | 2-5-8 Kazusakamatari Kisarazu-city Chiba 292-0818
| | Japan
4-3-3 | Date of deposit | 24 June 2008 (24.06.2008)
4-3-4 | Accession Number | NPMD NITE BP-597
4-5 | Designated States for Which
| Indications are Made | All designations

FOR RECEIVING OFFICE USE ONLY

0-4 | This form was received with the
| international application:
| (yes or no) |
0-4-1 | Authorized officer |

FOR INTERNATIONAL BUREAU USE ONLY

0-5 | This form was received by the
| International Bureau on: |
0-5-1 | Authorized officer |

## Claims

1. An imine reductase having the physicochemical properties of (1) to (5) below:
(1) activity: to generate (R)-2-methylpyrrolidine by reducing 2-methyl-1-pyrroline in a reduced nicotinamide adenine dinucleotide phosphate (hereinafter abbreviated as NADPH)-dependent manner;
(2) coenzyme dependency: use NADPH as a coenzyme;
(3) optimal pH: 6.0 to 7.0;
(4) optimal temperature: 40 to 55°C; and
(5) molecular weight: approximately 32,000 by sodium dodecyl sulfate polyacrylamide gel electrophoresis (hereinafter abbreviated as SDS-PAGE), and 65,000 to 70,000 by gel filtration.

2. The imine reductase of claim 1, which is derived from a microorganism belonging to the genus *Streptomyces.*

3. The imine reductase of claim 2, wherein the microorganism belonging to the genus *Streptomyces* is any one selected from the group consisting of
*Streptomyces* sp. NITE BP-594;
*Streptomyces* sp. NITE BP-595:
*Streptomyces* sp. NITE BP-596; and
*Streptomyces* sp. NITE BP-597.

4. The imine reductase of claim 2, wherein the microorganism belonging to the genus *Streptomyces* is any one selected from the group consisting of:
*Streptomyces griseorubiginosus;*
*Streptomyces microflavus;* and
*Streptomyces alboviridis.*

5. The imine reductase of claim 4, wherein *Streptomyces griseorubiginosus, Streptomyces microflavus,* and *Streptomyces alboviridis* are the following microbial strains:
*Streptomyces griseorubiginosus* NBRC 13047;
*Streptomyces microflavus* NBRC 13062; and
*Streptomyces alboviridis* NBRC 13013, respectively.

6. An imine reductase that has the physicochemical properties of (1) and (2) of claim 1, and which is encoded by the polynucleotide of any one of (a) to (e) below:
(a) a polynucleotide comprising the nucleotide sequence of SEQ ID NO: 5;
(b) a polynucleotide encoding a protein comprising the amino acid sequence of SEQ ID NO: 6;
(c) a polynucleotide encoding a protein comprising an amino acid sequence with a substitution, deletion, insertion, and/or addition of one or more amino acids in the amino acid sequence of SEQ ID NO: 6;
(d) a polynucleotide that hybridizes under a stringent condition to a DNA comprising the nucleotide sequence of SEQ ID NO: 5; and
(e) a polynucleotide encoding an amino acid sequence with 80% or higher sequence identity to the amino acid sequence of SEQ ID NO: 6.

7. An imine reductase that has the physicochemical properties of (1) and (2) of claim 1, which comprises a protein having the function of generating (R)-2-methylpyrrolidine with at least 80%ee or higher optical purity and is encoded by the polynucleotide of any one of (a) to (e) below:
(a) a polynucleotide comprising the nucleotide sequence of SEQ ID NO: 16;
(b) a polynucleotide encoding a protein comprising the amino acid sequence of SEQ ID NO: 17;
(c) a polynucleotide encoding a protein comprising an amino acid sequence with a substitution, deletion, insertion, and/or addition of one or more amino acids in the amino acid sequence of SEQ ID NO: 17;
(d) a polynucleotide that hybridizes under a stringent condition to a DNA comprising the nucleotide sequence of SEQ ID NO: 16; and
(e) a polynucleotide encoding an amino acid sequence that has 80% or higher sequence identity to the amino acid sequence of SEQ ID NO: 17.

8. A method for producing an optically active R-isomer amine derivative, which comprises the steps of:
contacting an imine derivative represented by formula (I) with at least one enzymatically active material selected from the group consisting of:
a culture of microorganism having the ability to stereoselectively reduce the compound; a microbial cell; and a processed product thereof; and
collecting the optically active R-isomer amine derivative represented by formula (II): 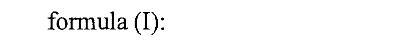 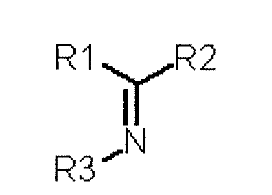 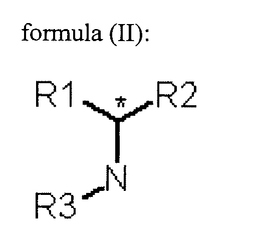
(wherein R1 and R2 groups each represents an alkyl group having one to three carbon atoms; R3 group represents a hydrogen or an alkyl group having one to three carbon atoms; and R1 and R3 together may form a ring).

9. The method of claim 8, wherein the compound of formula (I) is an imine derivative represented by formula (III) below and the compound of formula (II) is an amine derivative represented by formula (IV): 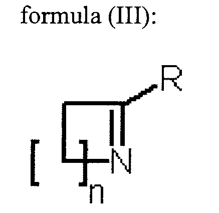 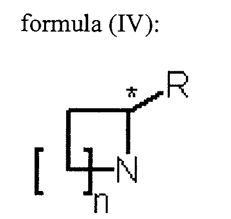 (wherein R group represents an alkyl group having one to three carbon atoms; and n represents an integer of 1 to 4).

10. The method of claim 9 for producing an optically active R-isomer amine derivative, wherein the compound represented by formula (III) is 2-methyl-1-pyrroline and the compound represented by formula (IV) is 2-methylpyrrolidine.

11. The method of any one of claims 8 to 10 for producing an optically active R-isomer amine derivative, wherein the microorganism belongs to the genus *Streptomyces.*

12. The method of claim 11 for producing an optically active R-isomer amine derivative, wherein the microorganism belonging to the genus *Streptomyces* is any one selected from the group consisting of:
*Streptomyces* sp. NITE BP-594;
*Streptomyces* sp. NITE BP-595;
*Streptomyces* sp. NITE BP-596; and
*Streptomyces* sp. NITE BP-597.

13. The method of claim 11 for producing an optically active R-isomer amine derivative, wherein the microorganism belonging to the genus *Streptomyces* is any one selected from the group consisting of:
*Streptomyces griseorubiginosus;*
*Streptomyces microflavus;* and
*Streptomyces alboviridis.*

14. The method of claim 13 for producing an optically active R-isomer amine derivative, wherein *Streptomyces griseorubiginosus, Streptomyces microflavus,* and *Streptomyces alboviridis* are the following microbial strains:
*Streptomyces griseorubiginosus* NBRC 13047;
*Streptomyces microflavus* NBRC 13062; and
*Streptomyces alboviridis* NBRC 13013, respectively.

15. A method for producing an optically active R-isomer amine derivative, which comprises the steps of:
contacting an imine derivative represented by formula (I) with at least one enzymatically active material selected from the group consisting of:
the imine reductase of any one of claims 1 to 7; a microorganism that produces the imine reductase; and a processed product thereof; and
collecting the optically active R-isomer amine derivative represented by formula (II): 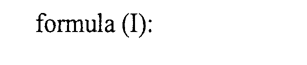 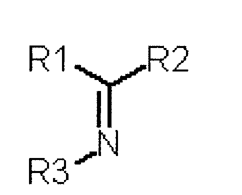 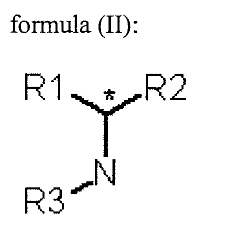
(wherein R1 and R2 groups each represents an alkyl group having one to three carbon atoms; R3 group represents a hydrogen or an alkyl group having one to three carbon atoms; and R1 and R3 together may form a ring).

16. The method of claim 15, wherein the compound represented by formula (I) is an imine derivative represented by formula (III) below and the compound of formula (II) is an amine derivative represented by formula (IV): 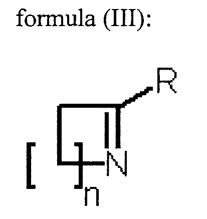 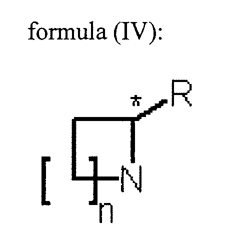 (wherein R group represents an alkyl group having one to three carbon atoms; and n represents an integer of 1 to 4).

17. A polynucleotide encoding an imine reductase that has the physicochemical properties of (1) and (2) of claim 1, which is any one of the following polynucleotides:
(a) a polynucleotide comprising the nucleotide sequence of SEQ ID NO: 5;
(b) a polynucleotide encoding a protein comprising the amino acid sequence of SEQ ID NO: 6;
(c) a polynucleotide encoding a protein comprising an amino acid sequence with a substitution, deletion, insertion, and/or addition of one or more amino acids in the amino acid sequence of SEQ ID NO: 6;
(d) a polynucleotide that hybridizes under a stringent condition to a DNA comprising the nucleotide sequence of SEQ ID NO: 5; and
(e) a polynucleotide encoding an amino acid sequence with 80% or higher sequence identity to the amino acid sequence of SEQ ID NO: 6.

18. A polynucleotide encoding an imine reductase that has the physicochemical properties of (1) and (2) of claim 1 and the function of generating (R)-2-methylpyrrolidine with at least 80%ee or higher optical purity, and is any one of the following polynucleotides:
(a) a polynucleotide comprising the nucleotide sequence of SEQ ID NO: 16;
(b) a polynucleotide encoding a protein comprising the amino acid sequence of SEQ ID NO: 17;
(c) a polynucleotide encoding a protein comprising an amino acid sequence with a substitution, deletion, insertion, and/or addition of one or more amino acids in the amino acid sequence of SEQ ID NO: 17;
(d) a polynucleotide that hybridizes under a stringent condition to a DNA comprising the nucleotide sequence of SEQ ID NO: 16; and
(e) a polynucleotide encoding an amino acid sequence with 80% or higher sequence identity to the amino acid sequence of SEQ ID NO: 17.

19. A recombinant vector inserted with the polynucleotide of claim 17 or 18.

20. The recombinant vector of claim 19, which is inserted additionally with a polynucleotide encoding a dehydrogenase that is capable of catalyzing a redox reaction using β-nicotinamide adenine dinucleotide phosphate as a coenzyme.

21. The vector of claim 20, wherein the dehydrogenase is a glucose dehydrogenase.

22. The recombinant vector of claim 21, wherein the glucose dehydrogenase is derived from *Bacillus subtilis.*

23. A transformant that maintains the polynucleotide of claim 17 or 18 or the vector of claim 19 in an expressible manner.

24. A method for producing a protein encoded by the polynucleotide of claim 17 or 18, which comprises the step of culturing the transformant of claim 23 and collecting an expression product.
